# EUROPEAN PATENT APPLICATION

(11) **EP 4 527 399 A1**
(43) Date of publication of application: **26.03.2025**
(21) Application number: 23807595.6
(22) Date of filing: 15.05.2023
(51) Int. Cl.: A61K 35/74, A61P 31/10, C12N 1/20, C12Q 1/18

(54) **COMPOSITION FOR TREATING RINGWORM**

(30) Priority: 16.05.2022 JP 2022080165
(71) Applicant: Maruho Co., Ltd., Osaka 531-0071 (JP)
(72) Inventor: HAYASHI, Naoki, Osaka-shi, Osaka 531-0071 (JP); KANAYAMA ,Shoji, Osaka-shi, Osaka 531-0071 (JP)
(74) Representative: Mewburn Ellis LLP
(86) International application number: PCT/JP2023/018046
(87) International publication number: WO 2023/223993

(57) **Abstract**

A novel composition for treating ringworm is provided.

A composition for treating ringworm, including at least one selected from the group consisting of a strain of Staphylococcus hominis, a strain of Staphylococcus haemolyticus, and a strain of Staphylococcus warneri.

## Description

### Technical Field

The present invention relates to a composition for treating ringworm, and the like.

### Background Art

Ringworm (Tinea) is a skin infection caused by fungi belonging to the genus Trichophyton (so-called Trichophyton). Ringworm is classified into tinea pedis (athlete's foot), tinea unguium (onychomycosis), tinea manus (ringworm of the hand), tinea corporis (body ringworm), tinea cruris (groin ringworm), tinea capitis (head ringworm), and the like depending on the onset site.

Ringworm is an infection caused by Trichophyton possessed by patients with ringworm. For example, in tinea pedis (athlete's foot) and tinea unguium (onychomycosis), which occur frequently, it is known that the major route of infection is Trichophyton present in swimming pools, public baths, restaurants, slippers, and the like.

Trichophyton is a non-indigenous fungus, and is parasitic on the keratin or subcutaneous tissue of the skin. When Trichophyton grows and invades into the stratum corneum, inflammation and blisters occur, and symptoms such as itch occur. Incidentally, Trichophyton uptakes the keratin, which is a main component of stratum corneum, as a nutrient source.

Conventionally, antifungal drugs such as itraconazole, miconazole, clotrimazole, ketoconazole, bifonazole, lanoconazole, luliconazole, efinaconazole, fosravuconazole, terbinafine, and butenafine have been used as therapeutic drugs for ringworm.

For example, Non Patent Literature 1 describes that tinea corporis (body ringworm) and tinea capitis (head ringworm) are frequently developed in pre-adolescent children, whereas tinea corporis (body ringworm), tinea cruris (groin ringworm), and tinea pedis (athlete's foot) are frequently developed in adolescents and adults, that diagnosis may be preferably performed by a potassium hydroxide preparation or culture because there are diseases exhibiting similar clinical symptoms, and that creams and oral preparations are appropriately selected as therapeutic agents.

In addition, Patent Literature 1 describes an invention related to an external preparation for treatment of tinea unguium, containing a) an antifungal active substance, b) a volatile component, c) a medium-chain fatty acid triglyceride, and d) ethyl lactate. According to the external preparation described in Patent Literature 1, it is described that when a medium-chain fatty acid triglyceride is used as a nonvolatile component and ethyl lactate is used as a permeation enhancer, the external preparation has excellent properties from the viewpoint of nail permeability, drug efficacy, and physical properties of the preparation.

Patent Literature 1 describes that an external preparation used for treating tinea unguium is excellent in terms of less systemic side effects and drug-drug interaction as compared with an oral preparation.

### Citation List

### Patent Literature

Patent Literature 1: WO 2019/088005 A

### Non Patent Literature

Non Patent Literature 1: American Family Physician, Volume 90, Number 10, 702-711.

### Summary of Invention

### Technical Problem

Under such circumstances, further development of a ringworm therapeutic agent has been conducted.

### Solution to Problem

The present invention provides, for example, a composition for treating ringworm of the following aspects.

[1] A composition for treating ringworm, including at least one selected from the group consisting of a strain of Staphylococcus hominis, a strain of Staphylococcus haemolyticus, and a strain of Staphylococcus warneri.
[2] The composition for treating ringworm according to [1], including the strain of Staphylococcus hominis.
[3] The composition for treating ringworm according to [2], wherein the strain of Staphylococcus hominis has 16s rDNA including a nucleotide sequence of SEQ ID NO: 1 or 16s rDNA including a nucleotide sequence having 95% or more of identity to SEQ ID NO: 1 and has an anti-ringworm action.
[4] The composition for treating ringworm according to [2] or [3], wherein the strain of Staphylococcus hominis includes at least one selected from the group consisting of Staphylococcus hominis ATCC27844, Staphylococcus hominis ATCC27845, Staphylococcus hominis ATCC700236, and Staphylococcus hominis A9 (ATCC Accession No.: PTA-125203).
[5] The composition for treating ringworm according to any one of [1] to [4], including the strain of Staphylococcus haemolyticus.
[6] The composition for treating ringworm according to [5], wherein the strain of Staphylococcus haemolyticus has a 16s rDNA including a nucleotide sequence of SEQ ID NO: 10 or a 16s rDNA including a nucleotide sequence having 95% or more of identity to SEQ ID NO: 10, and has an anti-ringworm action.
[7] The composition for treating ringworm according to [5] or [6], wherein the strain of Staphylococcus haemolyticus includes at least one selected from the group consisting of Staphylococcus haemolyticus ATCC29970, Staphylococcus haemolyticus ATCC700564, and Staphylococcus haemolyticus ATCC29969.
[8] The composition for treating ringworm according to any one of [1] to [7], including the strain of Staphylococcus warneri.
[9] The composition for treating ringworm according to [8], wherein the strain of Staphylococcus warneri has 16s rDNA including a nucleotide sequence of SEQ ID NO: 17 or 16s rDNA including a nucleotide sequence having 95% or more of identity to SEQ ID NO: 17, and has an anti-ringworm action.
[10] The composition for treating ringworm according to [8] or [9], wherein the strain of Staphylococcus warneri includes Staphylococcus warneri ATCC27836.
[11] The composition for treating ringworm according to any one of [1] to [10], wherein a causative fungus of the ringworm includes a fungus of the genus Trichophyton.
[12] The composition for treating ringworm according to [11], wherein the causative fungus of the ringworm includes at least one selected from the group consisting of a fungal strain of Trichophyton rubrum, a fungal strain of Trichophyton mentagrophytes, and a fungal strain of Trichophyton tonsurans.
[13] The composition for treating ringworm according to any one of [1] to [12], wherein the composition is an external preparation.
[14] A method for producing a composition for treating ringworm, the method including:
   a step (1) of collecting and culturing a bacterial flora from at least one of a malleolus and a foot sole of a human;
   a step (2) of isolating and culturing the bacterial flora cultured in the step (1) to obtain a candidate bacterium;
   a step (3) of evaluating an anti-ringworm fungal action of the candidate bacterium to identify a bacterium having an anti-ringworm fungal action; and
   a step (4) of preparing a composition for treating ringworm including the bacterium having an anti-ringworm fungal action;
   wherein the bacterium having an anti-ringworm fungal action includes at least one selected from the group consisting of a strain of Staphylococcus hominis, a strain of Staphylococcus haemolyticus, and a strain of Staphylococcus warneri.

### Advantageous Effects of Invention

According to the present invention, a novel composition for treating ringworm is provided. According to a preferred embodiment of the present invention, the present invention can be used even in a case where artificial chemical synthesis and extraction of a medicinal compound are difficult or in a case where the medicinal effect is based on a biological reaction derived from live bacteria, and the present invention can have one or more effects among prevention or reduction of side effects, prevention or suppression of skin rash around an affected area, a high skin colonization property, maintenance of remission, prevention of recurrence, reduction in the number of administrations, improvement in compliance, and a higher therapeutic effect.

### Brief Description of Drawings

Fig. 1 is a typical example of each evaluation of the anti-ringworm fungal action.
Fig. 2 is a diagram showing the results of anti-ringworm fungal action for each bacterial species of candidate bacteria (91 strains) obtained from the malleolus and the sole of the foot.
Fig. 3 is a phylogenetic tree of known bacteria and candidate bacteria belonging to the genus Staphylococcus.
Fig. 4 is a phylogenetic tree of known bacteria and candidate bacteria belonging to Staphylococcus hominis.
Fig. 5 is a phylogenetic tree of known bacteria and candidate bacteria of Staphylococcus haemolyticus.

### Description of Embodiments

Hereinafter, the present invention will be described in detail.

### <Composition for treating ringworm>

The composition for treating ringworm according to the present invention includes at least one selected from the group consisting of a strain of Staphylococcus hominis, a strain of Staphylococcus haemolyticus, and a strain of Staphylococcus warneri. The composition for treating ringworm according to the present invention can treat ringworm. In the present specification, the term "treatment" means to prevent or suppress the growth of a fungus belonging to the genus Trichophyton (so-called Trichophyton), or to provide a fungicidal action or a fungistatic action on the fungus.

It is known that there are individual differences in the onset and recurrence of ringworm. The present inventors predicted that the individual difference is in the skin indigenous bacteria, and examined the effects on Trichophyton of the skin indigenous bacteria collected from the malleolus (ankle) and the foot sole of a healthy human and the known strains of Staphylococcus. As a result, it was found that a strain of Staphylococcus hominis (hereinafter, also referred to as "S. hominis"), a strain of Staphylococcus haemolyticus (hereinafter, also referred to as "S. haemolyticus"), and a strain of Staphylococcus warneri (hereinafter, also referred to as "S. warneri"), which are skin indigenous bacteria, are effective for the treatment of ringworm. According to the present invention, a novel composition for treating ringworm can be provided.

Since the strain of S. hominis, the strain of S. haemolyticus, and the strain of S. warneri are skin indigenous bacteria, they have few or no side effects. Therefore, for example, the composition for treating ringworm can have an effect that it is possible to prevent or suppress skin rash around an affected area that can be caused by a conventional external preparation.

In addition, since the strain of S. hominis, the strain of S. haemolyticus, and the strain of S. warneri are skin indigenous bacteria, they are easily colonized on the skin. Therefore, for example, as compared with the conventional external preparation, the composition for treating ringworm can have an effect of prolonging the duration of the effect on the affected area of the skin, reducing the number of times of administration, and the like.

In addition, since the strain of S. hominis, the strain of S. haemolyticus, and the strain of S. warneri are live bacteria that are indigenous, the composition for treating ringworm can have a therapeutic effect even when artificial chemical synthesis and extraction of a medicinal compound are difficult, or when the medicinal effect is based on a biological reaction derived from live bacteria.

Therefore, the composition for treating ringworm according to the present invention can have effects such as less side effects, excellent compliance, and exhibiting a higher effect as compared with conventional external preparations.

### [Strain of Staphylococcus hominis]

In one embodiment, the composition for treating ringworm includes a strain of Staphylococcus hominis.

The strain of Staphylococcus hominis is a coagulase negative staphylococci (CNS) and is a gram-positive bacterium. The strain of S. hominis is a skin indigenous bacterium. It is presumed that the strain of S. hominis prevents or suppresses the growth or colonization of Trichophyton, or provides a fungicidal action or a fungistatic action on Trichophyton by direct or indirect damage to Trichophyton, competition of habitat, or the like.

The strain of S. hominis preferably includes a strain of S. hominis having an anti-ringworm fungal action. That is, in one preferred embodiment, a composition for treating ringworm including a strain of S. hominis having an anti-ringworm fungal action is provided. In the present specification, the term "anti-ringworm fungal action" means that the evaluation result of "++" or "+", preferably "++" is obtained when the anti-ringworm fungal action is evaluated by the method described in "4. Evaluation of anti-ringworm fungal action" of Examples. Therefore, a strain of S. hominis having an anti-ringworm fungal action can treat ringworm.

The strain of S. hominis is not particularly limited, but preferably has 16s rDNA including the nucleotide sequence of SEQ ID NO: 1 or 16s rDNA including a nucleotide sequence having 95% or more of identity to SEQ ID NO: 1, and has an anti-ringworm action. SEQ ID NO: 1 is a nucleotide sequence of a region between two common sequences commonly possessed by 16s rDNA of genus Staphylococcus as shown in Examples. At this time, among the two common sequences, the common sequence on the 5'-side is common sequence 1 (AGCTTGC), and the common sequence on the 3'-side is common sequence 2 (AAGCTGG), which are included in SEQ ID NO: 1. Here, for example, "16s rDNA including the nucleotide sequence of SEQ ID NO: 1" means that the nucleotide sequence of SEQ ID NO: 1 is present in the nucleotide sequence of 16s rDNA. In the present specification, the nucleotide sequences of SEQ ID NOs: 1 to 18 are specified by the method described in Examples. When one bacterium has two or more types of 16s rDNA, the 16s rDNA contained in the largest amount is defined as 16s rDNA of the bacterium. When the same number of two or more types of 16s rDNA is contained, the 16s rDNA having the nucleotide sequence having the highest identity among SEQ ID NO: 1 for the strain of S. hominis, SEQ ID NO: 10 for the strain of S. haemolyticus, and SEQ ID NO: 17 for the strain of S. warneri is defined as 16s rDNA of the bacterium.

The nucleotide sequence having 95% or more identity to SEQ ID NO: 1 preferably has 96% or more, 96.5% or more, 97% or more, 97.5% or more, 98% or more, 98.1% or more, 98.2% or more, 98.3% or more, 98.4% or more, 98.5% or more, 98.6% or more, 98.7% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more of identity to SEQ ID NO: 1.

Examples of the mutation of the nucleotide sequence having 95% or more of identity to SEQ ID NO: 1 include substitution, deletion, insertion, addition, and combinations thereof. In a preferred embodiment, the number of substitutions, deletions, insertions, additions, and combinations thereof is preferably 1 to 10, more preferably 1 to 5, and even more preferably 1 to 2.

The substitution is not particularly limited, but is preferably at least one substitution selected from the group consisting of guanine at position 45, thymine at position 139, thymine at position 140, adenine at position 309, and cytosine at position 1265, of SEQ ID NO: 1. In one embodiment, the guanine at position 45 can be substituted with adenine (45G>A (also expressed as G45 A)), the thymine at position 139 can be substituted with cytosine (139T>C), the thymine at position 140 can be substituted with cytosine (140 T>C), the adenine at position 309 can be substituted with cytosine (309A>C), and the cytosine at position 1265 can be substituted with thymine (1265C>T), of SEQ ID NO: 1.

The deletion is not particularly limited, but adenine at position 488 of SEQ ID NO: 1 may be deleted (488delA (also expressed as A488Δ)).

The insertion is not particularly limited, but can be inserted between positions 1182 and 1183 and between positions 1235 and 1236, of SEQ ID NO: 1. In one embodiment, adenine may be inserted between positions 1182 and 1183 (1182_1183insA (also expressed as Δ1183A)) and guanine may be inserted between positions 1235 and 1236 (1235_1236insG), of SEQ ID NO: 1.

In addition, the nucleotide sequence having 95% or more of identity to SEQ ID NO: 1 may be a nucleotide sequence possessed by a strain of S. hominis by natural mutation or the like, or a nucleotide sequence obtained by artificial mutation treatment. Examples of the artificial mutation treatment include methods such as genetic manipulation, genome editing, ultraviolet irradiation, radiation irradiation, ethyl methanesulfonate (MES) treatment, N-methyl-N-nitrosoguanidine (NTG) treatment, and nitrite treatment. These mutation treatments may be used alone or in combination of two or more thereof.

Examples of the nucleotide sequence having 95% or more of identity to SEQ ID NO: 1 include SEQ ID NOs: 2 to 9. The mutation sites of SEQ ID NOs: 2 to 9 are shown in Table 1 below.

**[Table 1]**

| SEQ ID NO. | Mutation site based on SEQ ID NO: 1 |
|---|---|
| SEQ ID NO: 1 | - |
| SEQ ID NO: 2 | 139T>C, 140T>C |
| SEQ ID NO: 3 | 1265C>T |
| SEQ ID NO: 4 | 140T>C |
| SEQ ID NO: 5 | 45G>A |
| SEQ ID NO: 6 | 45G>A, 309A>C |
| SEQ ID NO: 7 | 45G>A, 488delA |
| SEQ ID NO: 8 | 45G>A, 1182_1183insA |
| SEQ ID NO: 9 | 45G>A, 1235_1236insG |

The strain of S. hominis having 16s rDNA including the nucleotide sequence of SEQ ID NO: 1 is not particularly limited, and examples thereof include S. hominis ATCC27844 and S. hominis ATCC27845.

The strain of S. hominis having 16s rDNA including a nucleotide sequence having 95% or more of identity to SEQ ID NO: 1 and having an anti-ringworm action is not particularly limited, and examples thereof include S. hominis ATCC700236 and S. hominis A9 (ATCC Accession No.: PTA-125203).

In one embodiment, the strain of S. hominis is not particularly limited, but examples thereof include Staphylococcus hominis ATCC27844, Staphylococcus hominis ATCC27845, Staphylococcus hominis ATCC700236, Staphylococcus hominis FDAARGOS_748, Staphylococcus hominis J6, Staphylococcus hominis FDAARGOS_136, Staphylococcus hominis A9 (ATCC accession No. PTA-125203), Staphylococcus hominis C2, Staphylococcus hominis AMT2, Staphylococcus hominis AMT3, Staphylococcus hominis AMT4-C2, Staphylococcus hominis AMT4-G1, Staphylococcus hominis AMT4-D12. In addition to the above, as the strains belonging to S. hominis, strains described in "NCBI", [online], stored on February 17, 2022, [searched on February 17, 2022], and the Internet <https://www.ncbi.nlm.nih.gov/genome/browse/#!/prokaryotes/2014/> can be used. Among these, the strain belonging to S. hominis preferably includes at least one selected from the group consisting of S. hominis ATCC27844, S. hominis ATCC27845, S. hominis ATCC700236, S. hominis FDAARGOS_748, S. hominis J6, S. hominis FDAARGOS_136, and S. hominis A9, and more preferably includes at least one selected from the group consisting of S. hominis ATCC27844, S. hominis ATCC27845, S. hominis ATCC700236, and S. hominis A9. The strains belonging to S. hominis described above may be used alone or in combination of two or more thereof.

A strain of S. hominis having an anti-ringworm fungal action can be easily prepared by the following method. Specifically, a method for preparing a strain of S. hominis having an anti-ringworm fungal action includes: a step (1) of collecting and culturing a bacterial flora from at least one of a malleolus and a foot sole of a human (preferably, a healthy human who does not have a symptom of ringworm); a step (2) of isolating and culturing the bacterial flora cultured in the step (1) to obtain a candidate bacterium; and a step (3) of evaluating the anti-ringworm fungal action of the candidate bacterium to identify a bacterium having an anti-ringworm fungal action. Each step will be described later. The bacterial flora of the malleolus and foot sole of a human usually includes strains belonging to S. hominis known as skin indigenous bacteria. Then, according to the above method, a strain belonging to S. hominis having an anti-ringworm fungal action can be easily prepared.

The concentration of the strain of S. hominis in the composition for treating ringworm is preferably 10³ to 10¹¹ CFU/g, more preferably 10⁴ to 10¹⁰ CFU/g, and still more preferably 10⁵ to 10⁹ CFU/g. In the present specification, "CFU" means a colony forming unit. In the present specification, the concentration (CFU/g) of the bacterial strain in the composition for treating ringworm is calculated by diluting the composition for treating ringworm to an appropriate concentration, then plating the diluted solution on an agar medium for culturing bacteria, and measuring colonies appearing after culturing (see Clinical Microbiology Procedures Handbook volume 2

### THIRD EDITION).

The form of the strain of S. hominis in the composition for treating ringworm is not particularly limited, but is preferably a dry powder form (for example, lyophilized form, spray-dried form).

### [Strain of Staphylococcus haemolyticus]

In one embodiment, the composition for treating ringworm includes a strain of Staphylococcus haemolyticus.

The strain of Staphylococcus haemolyticus is a coagulase negative staphylococci (CNS) and is a gram-positive bacterium. Strains of S. haemolyticus are skin indigenous bacteria. It is presumed that the strain of S. haemolyticus also prevents or suppresses the growth of Trichophyton, or provides a fungicidal action or a fungistatic action on Trichophyton by direct or indirect damage to Trichophyton, competition of habitat, or the like.

The strain of S. haemolyticus preferably includes a strain of S. haemolyticus having an anti-ringworm fungal action. That is, in one preferred embodiment, a composition for treating ringworm including a strain of S. haemolyticus having an anti-ringworm fungal action is provided. A strain of S. haemolyticus having an anti-ringworm fungal action can treat ringworm.

The strain of S. haemolyticus is not particularly limited, but preferably has 16s rDNA including the nucleotide sequence of SEQ ID NO: 10 or 16s rDNA including a nucleotide sequence having 95% or more of identity to SEQ ID NO: 10, and has an anti-ringworm action. SEQ ID NO: 10 is a nucleotide sequence of a region between two common sequences commonly possessed by 16s rDNA of genus Staphylococcus as shown in Examples. At this time, among the two common sequences, the common sequence on the 5'-side is common sequence 1 (AGCTTGC), and the common sequence on the 3'-side is common sequence 2 (AAGCTGG), which are included in SEQ ID NO: 10. Here, for example, "16s rDNA including the nucleotide sequence of SEQ ID NO: 10" means that the nucleotide sequence of SEQ ID NO: 10 is present in the nucleotide sequence of 16s rDNA.

The nucleotide sequence having 95% or more identity to SEQ ID NO: 10 preferably has 96% or more, 96.5% or more, 97% or more, 97.5% or more, 98% or more, 98.1% or more, 98.2% or more, 98.3% or more, 98.4% or more, 98.5% or more, 98.6% or more, 98.7% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more of identity to SEQ ID NO: 10.

Examples of the mutation of the nucleotide sequence having 95% or more of identity to SEQ ID NO: 10 include substitution, deletion, insertion, addition, and combinations thereof. In a preferred embodiment, the number of substitutions, deletions, insertions, additions, and combinations thereof is preferably 1 to 10, more preferably 1 to 5, and even more preferably 1 to 2.

The substitution is not particularly limited, but is preferably at least one selected from the group consisting of cytosine at position 96, guanine at position 109, adenine at position 398, cytosine at position 952, and cytosine at position 1186, of SEQ ID NO: 10. In one embodiment, the cytosine at position 96 can be substituted with thymine (96C>T), the guanine at position 109 can be substituted with adenine (109G>A), the adenine at position 398 can be substituted with guanine (398A>G), the cytosine at position 952 can be substituted with thymine (952C>T), and the cytosine at position 1186 can be substituted with thymine (1186C>T), of SEQ ID NO: 10.

The deletion is not particularly limited, and the adenine at position 1210 (1210delA), the adenine at position 1219 (1219delA), the thymine at position 1225 (1225delT), and the adenine at position 1263 (1263delA), of SEQ ID NO: 10 may be deleted.

The insertion is not particularly limited, but it can be inserted between positions 1117 and 1118, between positions 1195 and 1196, and between positions 1234 and 1235, of SEQ ID NO: 10. In one embodiment, guanine may be inserted between positions 1117 and 1118 (1117_1118insG), cytosine may be inserted between positions 1195 and 1196 (1195_1196insC), and guanine may be inserted between positions 1234 and 1235 (1234_1235insG), of SEQ ID NO: 10.

The nucleotide sequence having 95% or more of identity to SEQ ID NO: 10 may be possessed by another strain of S. haemolyticus due to natural mutation or the like, or may be obtained by artificial mutation treatment, similarly to the strain of S. hominis. Examples of the artificial mutation treatment include methods such as genetic manipulation, genome editing, ultraviolet irradiation, radiation irradiation, ethyl methanesulfonate (MES) treatment, N-methyl-N-nitrosoguanidine (NTG) treatment, and nitrite treatment. These mutation treatments may be used alone or in combination of two or more thereof.

Examples of the nucleotide sequence having 95% or more of identity to SEQ ID NO: 10 include SEQ ID NOs: 11 to 16. The mutation sites of SEQ ID NOs: 11 to 16 are shown in Table 2 below.

**[Table 2]**

| SEQ ID NO. | Mutation site based on SEQ ID NO: 10 |
|---|---|
| SEQ ID NO: 10 | - |
| SEQ ID NO: 11 | 96C>T |
| SEQ ID NO: 12 | 109G>A |
| SEQ ID NO: 13 | 398A>G, 952C>T |
| SEQ ID NO: 14 | 398A>G |
| SEQ ID NO: 15 | 398A>G, 952C>T, 1186C>T |
| SEQ ID NO: 16 | 398A>G, 952C>T, 1117_1118insG, 1195_1196insC, 1210delA, 1219delA, 1225delT, 1234_1235insG, 1263delA |

The strain of S. haemolyticus having 16s rDNA including the nucleotide sequence of SEQ ID NO: 10 is not particularly limited, and examples thereof include S. haemolyticus ATCC29970 and S. haemolyticus ATCC700564.

In one embodiment, the strain of S. haemolyticus is not particularly limited, and examples thereof include Staphylococcus haemolyticus ATCC29970, Staphylococcus haemolyticus ATCC700564, Staphylococcus haemolyticus ATCC29969, Staphylococcus haemolyticus SH_12, and Staphylococcus haemolyticus JCSC1435. In addition to the above, as the strains belonging to S. haemolyticus, strains described in "NCBI", [online], stored on February 17, 2022, [searched on February 17, 2022], and the Internet <https://www.ncbi.nlm.nih.gov/genome/browse/#!/prokaryotes/1141/> can be used. Among these, the strain of S. haemolyticus preferably includes at least one selected from the group consisting of S. haemolyticus ATCC29970, S. haemolyticus ATCC700564, S. haemolyticus ATCC29969, S. haemolyticus SH_12, and S. haemolyticus JCSC1435, and more preferably includes at least one selected from the group consisting of Staphylococcus haemolyticus ATCC29970, Staphylococcus haemolyticus ATCC700564, and Staphylococcus haemolyticus ATCC29969. The strains of S. haemolyticus described above may be used alone or in combination of two or more thereof.

The strain of S. haemolyticus having an anti-ringworm fungal action can be easily prepared by the following method, similarly to the strain of S. hominis. Specifically, a method for preparing a strain of S. haemolyticus having an anti-ringworm fungal action includes: a step (1) of collecting and culturing a bacterial flora from at least one of a malleolus and a foot sole of a human (preferably, a healthy human who does not have a symptom of ringworm); a step (2) of isolating and culturing the bacterial flora cultured in the step (1) to obtain a candidate bacterium; and a step (3) of evaluating the anti-ringworm fungal action of the candidate bacterium to identify a bacterium having an anti-ringworm fungal action. Strains of S. haemolyticus with anti-ringworm fungal action may also be included in the bacterial flora of the malleolus and foot sole of a human. Therefore, according to the above method, a strain of S. haemolyticus having an anti-ringworm fungal action can be easily prepared.

The concentration of the strain of S. haemolyticus in the composition for treating ringworm is preferably 10³ to 10¹¹ CFU/g, more preferably 10⁴ to 10¹⁰ CFU/g, and still more preferably 10⁵ to 10⁹ CFU/g.

The form of the strain of S. haemolyticus in the composition for treating ringworm is not particularly limited, but is preferably a dry powder form (for example, lyophilized form, spray-dried form).

### [Staphylococcus warneri]

In one embodiment, the composition for treating ringworm includes a strain of Staphylococcus warneri.

The strain of Staphylococcus warneri is a coagulase negative staphylococci (CNS) and is a gram-positive bacterium. The strain of S. warneri is a skin resident bacterium. It is presumed that the strain of S. warneri also prevents or suppresses the growth of Trichophyton, or provides a fungicidal action or a fungistatic action on Trichophyton by direct or indirect damage to Trichophyton, competition of habitat, or the like.

The strain of S. warneri preferably includes a strain of S. warneri having an anti-ringworm fungal action. That is, in one preferred embodiment, a composition for treating ringworm including a strain of S. warneri having an anti-ringworm fungal action is provided. A strain of S. warneri having an anti-ringworm fungal action can treat ringworm.

The strain of S. warneri is not particularly limited, but preferably has 16s rDNA including the nucleotide sequence of SEQ ID NO: 17 or 16s rDNA including a nucleotide sequence having 95% or more of identity to SEQ ID NO: 17, and has an anti-ringworm action. SEQ ID NO: 17 is a nucleotide sequence of a region between two common sequences commonly possessed by 16s rDNA of genus Staphylococcus as shown in Examples. At this time, among the two common sequences, the common sequence on the 5'-side is common sequence 1 (AGCTTGC), and the common sequence on the 3'-side is common sequence 2 (AAGCTGG), which are included in SEQ ID NO: 17. Here, for example, "16s rDNA including the nucleotide sequence of SEQ ID NO: 17" means that the nucleotide sequence of SEQ ID NO: 17 is present in the nucleotide sequence of 16s rDNA.

The nucleotide sequence having 95% or more identity to SEQ ID NO: 17 preferably has 96% or more, 96.5% or more, 97% or more, 97.5% or more, 98% or more, 98.1% or more, 98.2% or more, 98.3% or more, 98.4% or more, 98.5% or more, 98.6% or more, 98.7% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more of identity to SEQ ID NO: 17.

Examples of the mutation of the nucleotide sequence having 95% or more of identity to SEQ ID NO: 17 include substitution, deletion, insertion, addition, and combinations thereof. In a preferred embodiment, the number of substitutions, deletions, insertions, additions, and combinations thereof is preferably 1 to 10, more preferably 1 to 5, and even more preferably 1 to 2.

The substitution is not particularly limited, but it is preferably cytosine at position 52 of SEQ ID NO: 17. In one embodiment, the cytosine at position 52 of SEQ ID NO: 17 may be substituted with guanine (52C>G).

The nucleotide sequence having 95% or more of identity to SEQ ID NO: 17 may be possessed by another strain of S. warneri due to natural mutation or the like, or may be obtained by artificial mutation treatment, similarly to the strain of S. hominis. Examples of the artificial mutation treatment include methods such as genetic manipulation, genome editing, ultraviolet irradiation, radiation irradiation, ethyl methanesulfonate (MES) treatment, N-methyl-N-nitrosoguanidine (NTG) treatment, and nitrite treatment. These mutation treatments may be used alone or in combination of two or more thereof.

Examples of the nucleotide sequence having 95% or more of identity to SEQ ID NO: 17 include SEQ ID NO: 18. The mutation site of SEQ ID NO: 18 is shown in Table 3 below.

**[Table 3]**

| SEQ ID NO. | Mutation site based on SEQ ID NO: 17 |
|---|---|
| SEQ ID NO: 17 | - |
| SEQ ID NO: 18 | 52C>G |

The strain of S. warneri having 16s rDNA including the nucleotide sequence of SEQ ID NO: 17 is not particularly limited, and examples thereof include S. warneri ATCC27836.

In one embodiment, the strain of S. warneri is not particularly limited, but includes S. warneri ATCC27836, S. warneri NCTC7291, S. warneri WB224, S. warneri 16A, S. warneri 22.1, and the like. In addition to the above, as the strains of S. warneri, strains described in "NCBI", [online], stored on February 17, 2022, [searched on February 17, 2022], and the Internet <https://www.ncbi.nlm.nih.gov/genome/browse/#!/prokaryotes/2073/> can be used. Among these, the strain of S. warneri preferably includes at least one selected from the group consisting of Staphylococcus warneri ATCC27836, S. warneri NCTC7291, S. warneri WB224, S. warneri 16A, S. warneri 22.1, and S. warneri WS479, and more preferably includes S. warneri ATCC27836. The strains of S. warneri described above may be used alone or in combination of two or more thereof.

The strain of S. warneri having an anti-ringworm fungal action can be easily prepared by the following method, similarly to the strain of S. hominis. Specifically, a method for preparing a strain of S. warneri having an anti-ringworm fungal action includes: a step (1) of collecting and culturing a bacterial flora from at least one of a malleolus and a foot sole of a human (preferably, a healthy human who does not have a symptom of ringworm); a step (2) of isolating and culturing the bacterial flora cultured in the step (1) to obtain a candidate bacterium; and a step (3) of evaluating the anti-ringworm fungal action of the candidate bacterium to identify a bacterium having an anti-ringworm fungal action. Strains of S. warneri with anti-ringworm fungal action may also be included in the bacterial flora of the malleolus and foot sole of a human. Therefore, according to the above method, a strain of S. warneri having an anti-ringworm fungal action can be easily prepared.

The concentration of the strain of S. warneri in the composition for treating ringworm is preferably 10³ to 10¹¹ CFU/g, more preferably 10⁴ to 10¹⁰ CFU/g, and still more preferably 10⁵ to 10⁹ CFU/g.

The form of the strain of S. warneri in the composition for treating ringworm is not particularly limited, but is preferably a dry powder form (for example, lyophilized form, spray-dried form).

### [Other strain of genus Staphylococcus]

The composition for treating ringworm may further contain other strains of the genus Staphylococcus. Here, the "other strain of the genus Staphylococcus" means a strain of the genus Staphylococcus other than S. hominis, S. haemolyticus, and S. warneri.

The other strains of the genus Staphylococcus are not particularly limited, and examples thereof include bacterial species having human isolation reports such as a strain of Staphylococcus saprophyticus, a strain of Staphylococcus kloosii, a strain of Staphylococcus lugdunensis, a strain of Staphylococcus capitis, a strain of Staphylococcus caprae, a strain of Staphylococcus epidermidis, and a strain of Staphylococcus aureus.

Examples of the strain of S. saprophyticus include those described in "NCBI", [online], stored on February 17, 2022, [searched on February 17, 2022], and the Internet <https://www.ncbi.nlm.nih.gov/genome/browse/#!/prokaryotes/1350/>.

Examples of the strain of S. kloosii include those described in "NCBI", [online], stored on February 17, 2022, [searched on February 17, 2022], and the Internet <https://www.ncbi.nlm.nih.gov/genome/browse/#!/prokaryotes/66884/>.

Examples of the strain of S. lugdunensis include those described in "NCBI", [online], stored on February 17, 2022, [searched on February 17, 2022], and the Internet <https://www.ncbi.nlm.nih.gov/genome/browse/#!/prokaryotes/2548/>.

Examples of the strain of S. capitis include those described in "NCBI", [online], stored on February 17, 2022, [searched on February 17, 2022], and the Internet <https://www.ncbi.nlm.nih.gov/genome/browse/#!/prokaryotes/2054/>.

Examples of the strain of S. caprae include those described in "NCBI", [online], stored on February 17, 2022, [searched on February 17, 2022], and the Internet <https://www.ncbi.nlm.nih.gov/genome/browse/#!/prokaryotes/1971/>.

Examples of the strain of S. epidermidis include those described in "NCBI", [online], stored on February 17, 2022, [searched on February 17, 2022], and the Internet <https://www.ncbi.nlm.nih.gov/genome/browse/#!/prokaryotes/155/>.

Examples of the strain of S. aureus include those described in "NCBI", [online], stored on February 17, 2022, [searched on February 17, 2022], and the Internet <https://www.ncbi.nlm.nih.gov/genome/browse/#!/prokaryotes/154/>.

From the viewpoint of obtaining a higher ringworm therapeutic effect, the other strain of the genus Staphylococcus preferably includes at least one selected from the group consisting of a strain of S. capitis, a strain of S. caprae, a strain of S. epidermidis, a strain of Staphylococcus kloosii, a strain of Staphylococcus lugdunensis, and a strain of Staphylococcus saprophyticus, more preferably includes at least one selected from the group consisting of a strain of S. capitis and a strain of S. caprae, and particularly preferably includes at least one of S. capitis ATCC27840 and S. caprae ATCC35538. In addition, from the viewpoint of adjusting the balance of the skin indigenous bacteria in the affected area to which the composition for treating ringworm is applied, the other strain of the genus Staphylococcus preferably includes a strain of S. epidermidis, and more preferably includes at least one of S. epidermidis ATCC12228 and S. epidermidis ATCCC14990. These other strains of the genus Staphylococcus may be used alone or in combination of two or more thereof.

The concentration of other strains of the genus Staphylococcus in the composition for treating ringworm is not particularly limited, but is preferably 10³ to 10¹¹ CFU/g, more preferably 10⁴ to 10¹⁰ CFU/g, and still more preferably 10⁵ to 10⁹ CFU/g. When two or more other strains of the genus Staphylococcus are contained in combination, it is preferable that two or more other strains of the genus Staphylococcus fall within the above range for each of the bacterial species.

The form of the other Staphylococcus strain in the composition for treating ringworm is not particularly limited, but is preferably a dry powder form (for example, lyophilized form, spray-dried form). The form of the other strain of the genus Staphylococcus is preferably the same form as at least one selected from the group consisting of the strain of S. hominis, the strain of S. haemolyticus, and the strain of S. warneri described above.

### [Strain of other genera]

The composition for treating ringworm may further contain a strain of another genus. Here, the "strain of another genus" means a bacterial strain other than the strain of the genus Staphylococcus.

The strain of the other genera is not particularly limited, but is preferably a skin indigenous bacterium (that is, skin indigenous bacteria other than Staphylococcus bacteria), and examples thereof include a strain of Cutibacterium acnes, a strain of Streptococcus pyogenes (Group A β-hemolytic streptococci), a strain of Pseudomonas aeruginosa, a strain of Escherichia coli, a strain of Bacillus cereus, a strain of Enterococcus faecalis, and a strain of Moraxella osloensis.

Examples of the strain of Cutibacterium acnes include those described in "NCBI", [online], stored on February 17, 2022, [searched on February 17, 2022], and the Internet <https://www.ncbi.nlm.nih.gov/genome/browse/#!/prokaryotes/1140/>.

Examples of the strain of Streptococcus pyogenes (group A β-hemolytic streptococci) include those described in "NCBI", [online], stored on February 17, 2022, [searched on February 17, 2022], and the Internet <https://www.ncbi.nlm.nih.gov/genome/browse/#!/prokaryotes/175/>.

Examples of the strain of Pseudomonas aeruginosa include those described in "NCBI", [online], stored on February 17, 2022, [searched on February 17, 2022], and the Internet <https://www.ncbi.nlm.nih.gov/genome/browse/#!/prokaryotes/187/>.

Examples of the strain of Escherichia coli include those described in "NCBI", [online], stored on February 17, 2022, [searched on February 17, 2022], and the Internet <https://www.ncbi.nlm.nih.gov/genome/browse/#!/prokaryotes/167/>.

Examples of the strain of Bacillus cereus include those described in "NCBI", [online], stored on February 17, 2022, [searched on February 17, 2022], and the Internet <https://www.ncbi.nlm.nih.gov/genome/browse/#!/prokaryotes/157/>.

Examples of the strain of Enterococcus faecalis include those described in "NCBI", [online], stored on February 17, 2022, [searched on February 17, 2022], and the Internet <https://www.ncbi.nlm.nih.gov/genome/browse/#!/prokaryotes/808/>.

Examples of the strain of Moraxella osloensis include those described in "NCBI", [online], stored on February 17, 2022, [searched on February 17, 2022], and the Internet <https://www.ncbi.nlm.nih.gov/genome/browse/#!/prokaryotes/12468/>.

Among these, from the viewpoint of obtaining a higher therapeutic effect for ringworm, the strain of other genera preferably includes at least one selected from the group consisting of a strain of Pseudomonas aeruginosa, a strain of Escherichia coli, a strain of Bacillus cereus, a strain of Enterococcus faecalis, and a strain of Moraxella osloensis, more preferably includes at least one selected from the group consisting of a strain of Pseudomonas aeruginosa, a strain of Escherichia coli, and a strain of Enterococcus faecalis, and still more preferably includes at least one selected from the group consisting of Pseudomonas aeruginosa ATCC27853, Escherichia coli ATCC25922, and Enterococcus faecalis ATCC29212. From the viewpoint of adjusting the balance of the skin indigenous bacteria in the affected area to which the composition for treating ringworm is applied, the strain of other genera preferably includes at least one selected from the group consisting of a strain of Cutibacterium acnes, a strain of Streptococcus pyogenes (group A β-hemolytic streptococci), and a strain of Pseudomonas aeruginosa, more preferably includes a strain of Cutibacterium acnes, and still more preferably includes Cutibacterium acnes ATCC6919. These strains of other genera may be used alone or in combination of two or more thereof.

The concentration of the strain of other genera in the composition for treating ringworm is not particularly limited, but is preferably 10³ to 10¹¹ CFU/g, more preferably 10⁴ to 10¹⁰ CFU/g, and still more preferably 10⁵ to 10⁹ CFU/g. When two or more other strains of other genera are contained in combination, it is preferable that two or more other strains of other genera fall within the above range for each of the bacterial species.

The form of the strain of other genera in the composition for treating ringworm is not particularly limited, but is preferably a dry powder form (for example, lyophilized form, spray-dried form). The form of the strain of other genera is preferably the same form as at least one selected from the group consisting of the strain of S. hominis, the strain of S. haemolyticus, and the strain of S. warneri described above.

### [Antifungal agent]

The composition for treating ringworm may further contain an antifungal agent. By containing an antifungal agent, the therapeutic effect of ringworm can be enhanced.

The antifungal agent is not particularly limited, and examples thereof include itraconazole, miconazole, clotrimazole, ketoconazole, bifonazole, lanoconazole, luliconazole, efinaconazole, fosravuconazole, terbinafine, and butenafine. These antifungal agents may be used alone or in combination of two or more thereof.

The concentration of the antifungal agent in the composition for treating ringworm is not particularly limited, but is preferably 0.1 to 30 mass% and more preferably 0.5 to 15 mass% with respect to the total mass of the composition for treating ringworm.

### [Additive]

The composition for treating ringworm may contain an additive. The additive is not particularly limited, and examples thereof include a base, a wetting agent, a thickener, an emulsifier, an emulsifying aid, a preservative, a stabilizer, and a pH adjuster.

### (Base)

Examples of the base include a hydrophobic base, a hydrophilic base, and water. In the present specification, the "base" means an additive having a content of 40 mass% or more, preferably 50 to 99 mass% with respect to the total mass of the composition for treating ringworm.

The hydrophobic base is not particularly limited, but examples thereof include a higher hydrocarbon such as squalane, liquid paraffin, light liquid paraffin, petrolatum, ceresin wax, microcrystalline wax, squalene, or gelled hydrocarbon; oils such as olive oil, jojoba oil, sesame oil, soybean oil, cocoa butter, camellia oil, peanut oil, beef tallow, lard, triacetin, and hydrogenated castor oil; waxes such as beeswax, white beeswax, carnauba wax, and lanolin; fatty acids such as stearic acid and oleic acid; higher alcohols such as lanolin alcohol, myristyl alcohol, cetanol (cetyl alcohol), stearyl alcohol, cetostearyl alcohol, and cholesterol; and fatty acid esters such as isopropyl myristate, stearyl myristate, and medium-chain fatty acid triglyceride.

The hydrophilic base is not particularly limited, but may be a lower alcohol such as ethanol, propanol, or isopropanol; polyhydric alcohol such as glycerin, 1,3-butylene glycol, and propylene glycol; sugar alcohols such as sorbitol and mannitol; and macrogol.

The above-mentioned bases may be used alone or in combination of two or more thereof.

### (Wetting agent)

The wetting agent keeps the skin moist when the composition for treating ringworm is applied.

The wetting agent is not particularly limited, and examples thereof include petrolatum, glycerin, propylene glycol, and 1,3-butylene glycol.

The above-mentioned wetting agents may be used alone or in combination of two or more thereof.

### (Thickener)

The thickener increases the viscosity or gels the composition for treating ringworm.

The thickener is not particularly limited, and examples thereof include gelatin, agar, carrageenan, gum arabic, tragacanth, sodium alginate, propylene glycol alginate, carboxyvinyl polymer, polyvinyl alcohol (partially saponified product), sodium alginate, methyl cellulose, carboxymethyl cellulose, water-soluble cellulose derivatives (hydroxyethyl cellulose, hydroxypropyl cellulose, hydroxypropyl methyl cellulose, hydrophobized hydroxypropyl methyl cellulose, and the like), sodium polyacrylate, glycerin monooleate, pectin, and xanthan gum.

The above-mentioned thickeners may be used alone or in combination of two or more thereof.

### (Emulsifier)

The emulsifier emulsifies the composition for treating ringworm into an oil-in-water (o/w) form or a water-in-oil (w/o) form.

The emulsifier is not particularly limited, but examples thereof include cationic surfactants such as alkylamine salts, alkylamine polyoxyethylene adducts, fatty acid triethanolamine monoester salts, acylaminoethyldiethylamine salts, and fatty acid polyamine condensates; anionic surfactants such as polyoxyethylene alkyl ether phosphates, alkyl sulfates, saturated higher fatty acid salts, and N-acyl amino acid salts; nonionic surfactants such as sucrose fatty acid ester, glycerin fatty acid ester, polyglycerin fatty acid ester, polyoxyethylene fatty acid ester, polyoxyethylene sorbitan fatty acid ester, polyoxyethylene castor oil, polyoxyethylene hydrogenated castor oil, polyethylene glycol, polyethylene glycol fatty acid ester, polyoxyethylene polyoxypropylene glycol, and polyoxyethylene alkyl ether; amphoteric surfactants such as alkyl betaine, alkyl amide betaine, alkyl sulfobetaine, imidazoline, lauryldimethylaminoacetic acid betaine, and alkyldiaminoethylglycine.

The above-mentioned emulsifiers may be used alone or in combination of two or more thereof.

### (Emulsifying aid)

The emulsifying aid stabilizes emulsification when emulsifying the composition for treating ringworm into an oil-in-water (o/w) type or a water-in-oil (w/o) type.

The emulsifying aid is not particularly limited, and examples thereof include cetanol (cetyl alcohol), stearyl alcohol, oleyl alcohol, and isostearyl alcohol.

The emulsifying aids may be used alone or in combination of two or more thereof.

### (Preservative)

The preservative prevents or suppresses contamination and degradation of the composition for treating ringworm due to foreign microorganisms.

The preservative is not particularly limited, and examples thereof include paraoxybenzoic acid, methyl paraoxybenzoate, ethyl paraoxybenzoate, propyl paraoxybenzoate, butyl paraoxybenzoate, sodium benzoate, chlorobutanol, chlorocresol, benzyl alcohol, salicylic acid, phenoxyethanol, thymol, dibutylhydroxytoluene, sodium edetate hydrate, sodium dehydroacetate, sorbic acid, potassium sorbate, and benzalkonium chloride.

The above-mentioned preservatives may be used alone or in combination of two or more thereof.

### (Stabilizer)

The stabilizer prevents or suppresses killing, degradation, and physical changes of components contained in the composition for treating ringworm.

The stabilizer is not particularly limited, and examples thereof include sodium bisulfite, sodium pyrosulfite, ascorbic acid, tocopherol, dibutylhydroxytoluene, sodium edetate (EDTA), and benzotriazole.

The above-mentioned stabilizer may be used alone or in combination of two or more thereof.

### (pH adjusting agent)

The pH adjusting agent maintains stability of components contained in the composition for treating ringworm, and prevents or reduces irritation to a living body of the composition for treating ringworm.

The pH adjusting agent is not particularly limited, and examples thereof include lactic acid, acetic acid, acetates (sodium acetate, etc.), citric acid, citrates (sodium citrate, etc.), phosphoric acid, phosphates (sodium phosphate, etc.), diisopropanolamine, triisopropanolamine, triethanolamine, potassium hydroxide, sodium hydroxide, etc.

These pH adjusting agents may be used alone or in combination of two or more thereof.

### [Dosage form]

The dosage form of the composition for treating ringworm is not particularly limited, but is preferably an external preparation. When the composition for treating ringworm is used as an external preparation, at least one selected from the group consisting of a strain of S. hominis, a strain of S. haemolyticus, and a strain of S. warneri can be colonized on the skin, and it is possible to effectively prevent or suppress the growth and colonization of Trichophyton, or to provide a fungicidal action or a fungistatic action on Trichophyton.

The specific form of the external preparation is not particularly limited, and examples thereof include ointments, creams, gels, and lotions.

The ointment is a semi-solid external preparation in which an active ingredient to be applied to the skin is dissolved or dispersed in a base, and examples thereof include an oil-and-fat ointment and a water-soluble ointment. The cream is a semi-solid external preparation emulsified in an oil-in-water (o/w) type or a water-in-oil (w/o) type to be applied to the skin. The gel agent is a gel-like external preparation applied to the skin, and examples thereof include an aqueous gel agent and an oily gel agent. The lotion is a liquid external preparation in which an active ingredient (strain of S. hominis, strain of S. haemolyticus, strain of S. warneri, etc.) is dissolved or dispersed in an aqueous solution.

### [Trichophyton]

The causative fungus of the ringworm is Trichophyton, specifically Trichophyton fungi. Examples of the Trichophyton include a fungal strain of Trichophyton rubrum, a fungal strain of Trichophyton mentagrophytes, and a fungal strain of Trichophyton tonsurans.

Examples of the fungal strain of Trichophyton rubrum include Trichophyton rubrum ATCC28188 and Trichophyton rubrum ATCC22402.

Examples of the fungal strain of Trichophyton mentagrophytes include Trichophyton mentagrophytes TIMM2789 and Trichophyton mentagrophytes ATCCMYA-4439.

Examples of the fungal strain of Trichophyton tonsurans include Trichophyton tonsurans ATCC56186 and Trichophyton tonsurans ATCC28942.

The causative fungus of the ringworm preferably includes a fungus of the genus Trichophyton, more preferably includes at least one selected from the group consisting of a fungal strain of Trichophyton rubrum, a fungal strain of Trichophyton mentagrophytes, and a fungal strain of Trichophyton tonsurans, further preferably includes a fungal strain of Trichophyton rubrum, and particularly preferably includes Trichophyton rubrum ATCC28188. The causative fungus of the ringworm may be one kind or two or more kinds.

Ringworm is developed by the Trichophyton. Note that ringworm is classified into tinea pedis (athlete's foot), tinea unguium (onychomycosis), tinea manus (ringworm of the hand), tinea corporis (body ringworm), tinea cruris (groin ringworm), tinea capitis (head ringworm), and the like depending on the onset site. Since the skin permeability of the external preparation varies depending on the onset site, it is preferable to appropriately prepare the composition of the external preparation depending on the onset site.

### [Administration method]

The method for administering the composition for treating ringworm is not particularly limited, but when the composition for treating ringworm is an external preparation, the composition is applied to an affected area.

The number of administrations (the number of times of application) is not particularly limited, and may be any of three times a day, twice a day, once a day, once every 2 days, once every 3 days, once every 4 days, once every 5 days, once every 6 days, once every 7 days, and the like. Among them, the number of administrations is preferably once a day, once every 2 days, once every 3 days, once every 4 days, once every 5 days, once every 6 days, and once every 7 days. Since the composition for treating ringworm according to the present invention contains at least one selected from the group consisting of a strain of S. hominis, a strain of S. haemolyticus, and a strain of S. warneri, which are excellent in skin colonization properties, as compared with conventional ringworm therapeutic agents, it is possible to maintain remission, prevent recurrence, and the like by exerting a sustained effect associated with the colonization of live bacteria, and it is possible to reduce the number of administrations and to increase patient compliance.

When the composition for treating ringworm is an external preparation, the application amount is preferably 0.1 to 180 mg/cm², and more preferably 1 to 10 mg/cm². At this time, the application amount per one application of at least one selected from the group consisting of a strain of S. hominis, a strain of S. haemolyticus, and a strain of S. warneri in the composition for treating ringworm is preferably 10⁰ to 10⁸ CFU/cm², more preferably 10¹ to 10⁷ CFU/cm², and still more preferably 10² to 10⁶ CFU/cm². When two or more of a strain of S. hominis, a strain of S. haemolyticus, and a strain of S. warneri are contained in the composition for treating ringworm, the total amount thereof is preferably in the above range.

The affected area to which the composition for treating ringworm is applied is not particularly limited, but is usually an affected area having a symptom of ringworm. Since the composition for treating ringworm according to the present invention contains at least one selected from the group consisting of a strain of S. hominis, a strain of S. haemolyticus, and a strain of S. warneri, which are skin indigenous bacteria, the number of administrations can be reduced as compared with the conventional external preparation due to high skin colonization properties, and the problem of skin rash around the affected area does not occur or hardly occurs, and the compliance of the patient can be increased.

The above-described composition for treating ringworm exhibits a therapeutic effect by use alone, but may be combined with other therapeutic agents.

Examples of the other therapeutic agent include an internal preparation and an external preparation. At this time, the internal preparation or the external preparation contains at least one of antifungal drugs such as itraconazole, miconazole, clotrimazole, ketoconazole, bifonazole, lanoconazole, luliconazole, efinaconazole, fosravuconazole, terbinafine, and butenafine. The above-mentioned other therapeutic agents may be used in combination of two or more thereof.

That is, according to an embodiment of the present invention, a combination agent of a composition for treating ringworm and other therapeutic agents is provided. At this time, when the composition for treating ringworm is an external preparation, the other therapeutic agent is preferably an internal preparation. In addition, the composition for treating ringworm and other therapeutic agents may be administered simultaneously or sequentially.

### <Method for producing composition for treating ringworm>

According to an embodiment of the present invention, a method for producing a composition for treating ringworm is provided. The producing method includes: a step (1) of collecting and culturing a bacterial flora from at least one of a malleolus and a foot sole of a human (preferably, a healthy human who does not have a symptom of ringworm); a step (2) of isolating and culturing the bacterial flora cultured in the step (1) to obtain a candidate bacterium; a step (3) of evaluating the anti-ringworm fungal action of the candidate bacterium to identify a bacterium having an anti-ringworm fungal action; and a step (4) of preparing a composition for treating ringworm including the bacterium having the anti-ringworm fungal action. In this case, the bacteria having an anti-ringworm fungal action include at least one selected from the group consisting of a strain of S. hominis, a strain of S. haemolyticus, and a strain of S. warneri. Note that the strain of S. hominis is preferably a strain of S. hominis having an anti-ringworm fungal action. In addition, the strain of S. haemolyticus is preferably a strain of S. haemolyticus having an anti-ringworm fungal action. In addition, the strain of S. warneri is preferably a strain of S. warneri having an anti-ringworm fungal action. Specific examples of the strain of S. hominis, the strain of S. haemolyticus, and the strain of S. warneri are as described above.

### [Step (1)]

The step (1) is a step of collecting and culturing a bacterial flora from at least one of the malleolus and the foot sole of a human (preferably a healthy human who does not have the symptom of ringworm). The bacterial flora is collected from a malleolus and/or foot sole of a human using a swab or the like, and is plated on a culture medium and cultured. Thereby, colonies are formed for each strain.

### [Step (2)]

The step (2) is a step of isolating and culturing the bacterial flora cultured in the step (1) to obtain candidate bacteria. Colonies formed by the culture in the step (1) are each collected with a platinum loop or the like, plated on a culture medium, and isolated and cultured. This makes it possible to isolate a large number of bacteria contained in the bacterial flora contained in the malleolus and/or foot sole of the human. Each of the cultured bacteria is a candidate bacterium, and it is preferable that the bacteria is suspended in a bacterial stock solution and stored as a skin bacteria library at a low temperature (for example, -100 to -50°C, preferably -90 to -70°C).

### [Step (3)]

The step (3) is a step of evaluating the anti-ringworm fungal action of the candidate bacteria obtained in the step (2) to identify bacteria having the anti-ringworm fungal action.

In the step (3), first, among candidate bacteria isolated from the malleolus and/or foot sole of the human, which candidate bacteria have an anti-ringworm fungal action is evaluated. At this time, the evaluation of the anti-ringworm fungal action of the candidate bacteria is performed by the method described in "4. Evaluation of anti-ringworm fungal action" of Examples.

Next, bacteria having an anti-ringworm fungal action are selected, and the bacteria are identified. The identification method is not particularly limited, and examples thereof include a method of comparing 16s rDNA sequences, a method of creating a phylogenetic tree, and the like. For example, in the method by comparing 16s rDNA sequences, bacteria can be identified by analyzing 16s rDNA sequences of bacteria of interest and performing BLAST search of NCBI using the DNA sequence data. In addition, in the method for creating a phylogenetic tree, a 16s rDNA sequence of bacteria is analyzed, and 16s rDNA sequence data of known bacteria is also analyzed to create a phylogenetic tree, and bacteria of interest can be identified from the phylogenetic tree. Note that the step (3) can include a step of calculating the identity of the 16s rDNA sequence of the bacteria of interest to SEQ ID NOs: 1, 10, 17, and the like, a step of confirming the DNA sequence identity of the bacteria of interest, and the like when the bacteria of interest are identified as a strain of S. hominis, a strain of S. haemolyticus, or a strain of S. warneri.

The bacteria having the anti-ringworm fungal action identified by step (3) may include at least one selected from the group consisting of a strain of S. hominis having an anti-ringworm fungal action, a strain of S. haemolyticus having an anti-ringworm fungal action, and a strain of S. warneri having an anti-ringworm fungal action. Therefore, by extracting at least one selected from the group consisting of a strain of S. hominis having an anti-ringworm fungal action, a strain of S. haemolyticus having an anti-ringworm fungal action, and a strain of S. warneri having an anti-ringworm fungal action, of interest, from the stored skin bacterial library, a strain of S. hominis having an anti-ringworm fungal action, a strain of S. haemolyticus having an anti-ringworm fungal action, and a strain of S. warneri having an anti-ringworm fungal action can be obtained.

### [Step (4)]

The step (4) is a step of preparing a composition for treating ringworm including bacterium having an anti-ringworm fungal action. Bacteria having an anti-ringworm fungal action specified in step (3) are appropriately cultured, purified, freeze-dried, and the like to prepare a composition for treating ringworm including bacteria having an anti-ringworm fungal action. The bacteria having an anti-ringworm fungal action include at least one selected from the group consisting of a strain of S. hominis, a strain of S. haemolyticus, and a strain of S. warneri, and preferably include at least one selected from the group consisting of a strain of S. hominis having an anti-ringworm fungal action, a strain of S. haemolyticus having an anti-ringworm fungal action, and a strain of S. warneri.

### <Treatment method>

According to one aspect of the present invention, a method for treating ringworm is provided. At this time, the treatment method includes applying the composition for treating ringworm described above to an affected area. The composition for treating ringworm, the administration method thereof, and the like are as described above.

### Examples

Hereinafter, the present invention will be specifically described with reference to Examples, but the present invention is not limited thereto.

### 1. Collection of candidate bacteria

Bacterial flora was collected using a swab from each one site of the malleolus and the foot sole of five healthy humans. The swab to which the bacterial flora was attached was plated on Anaero Columbia RS Blood Agar Medium, and the swab was statically cultured for 1 day under aerobic conditions at 35°C. After culturing, characteristic colonies in terms of morphology, color, degree of dryness, and the like were collected with a platinum loop.

The platinum loop to which the collected colonies adhered was plated on Anaero Columbia RS Blood Agar Medium, and isolated and cultured for 1 day under aerobic conditions at 35°C.

After culturing, the grown colony groups were collected with platinum loop and suspended in 25% glycerol bacterial stock solution and stored at -80°C. The collected candidate bacteria were 91 strains in total. At this time, 52 strains of candidate bacteria were obtained from the malleolus, and 39 strains of candidate bacteria were obtained from the foot sole.

### 2. Preparation of Known Bacteria

The following 32 strains of bacteria were prepared as known bacteria.
(1) Acinetobacter baumannii ATCC17978
(2) Corynebacterium glaucum JCM12208
(3) Corynebacterium jeikeium ATCC43734
(4) Corynebacterium striatum NBRC15291
(5) Enterococcus faecalis ATCC29212
(6) Escherichia coli ATCC25922
(7) Micrococcus luteus ATCC4698
(8) Moraxella atlantae NBRC14588
(9) Neisseria gonorrhoeae ATCC19424
(10) Pseudomonas aeruginosa ATCC27853
(11) Staphylococcus aureus ATCC29213
(12) Staphylococcus epidermidis ATCC12228
(13)Staphylococcus haemolyticus ATCC29970
(14)Staphylococcus hominis ATCC27844
(15)Streptococcus agalactiae ATCC13813
(16) Streptococcus mitis JCM12971
(17) Streptococcus pyogenes ATCC12344
(18) Staphylococcus aureus ATCC12600
(19) Staphylococcus epidermidis ATCC14990
(20) Staphylococcus capitis ATCC27840
(21) Staphylococcus caprae ATCC35538
(22) Staphylococcus cohnii ATCC29974
(23) Staphylococcus haemolyticus ATCC700564
(24) Staphylococcus haemolyticus ATCC29969
(25) Staphylococcus hominis ATCC27845
(26) Staphylococcus hominis ATCC700236
(27) Staphylococcus kloosii ATCC43959
(28) Staphylococcus lentus ATCC700403
(29) Staphylococcus lugdunensis ATCC49576
(30) Staphylococcus lugdunensis ATCC43809
(31) Staphylococcus warneri ATCC27836
(32) Staphylococcus saprophyticus ATCC15305

### 3. Preparation of culture medium for evaluation

In order to evaluate the anti-ringworm fungal action of candidate bacteria and known bacteria, a fungal mixed culture medium containing Trichophyton was prepared as an evaluation medium.

Specifically, one bead of Trichophyton (Trichophyton rubrum ATCC28188) stored in Microbank was taken, plated on a Potato dextrose agar (PDA) slant agar medium, and statically cultured at 30°C under aerobic conditions.

After the culturing, phosphate buffered saline (PBS) containing 0.05% polysorbate 80 was added to the PDA slant agar medium to prepare a fungal inoculum solution.

PDA was dissolved in ultrapure water to a concentration of 39.0 g/L, and the resulting solution was heated in an autoclave (121°C, 20 min). After air cooling, the fungal inoculum solution was mixed with the PDA lysate, and the mixture was dispensed into a petri dish and solidified to prepare a fungal mixed culture medium.

### 4. Evaluation of anti-ringworm fungal action

Using a fungal mixed culture medium as an evaluation medium, the anti-ringworm action of candidate bacteria (91 strains) and known bacteria (32 strains) was evaluated.

Specifically, one bead containing candidate bacteria or known bacteria stored in Microbank was taken, plated on Anaero Columbia RS Blood Agar Medium, and statically cultured under aerobic conditions at 35°C for 1 to 2 days, and the resulting bacteria were collected with a platinum loop.

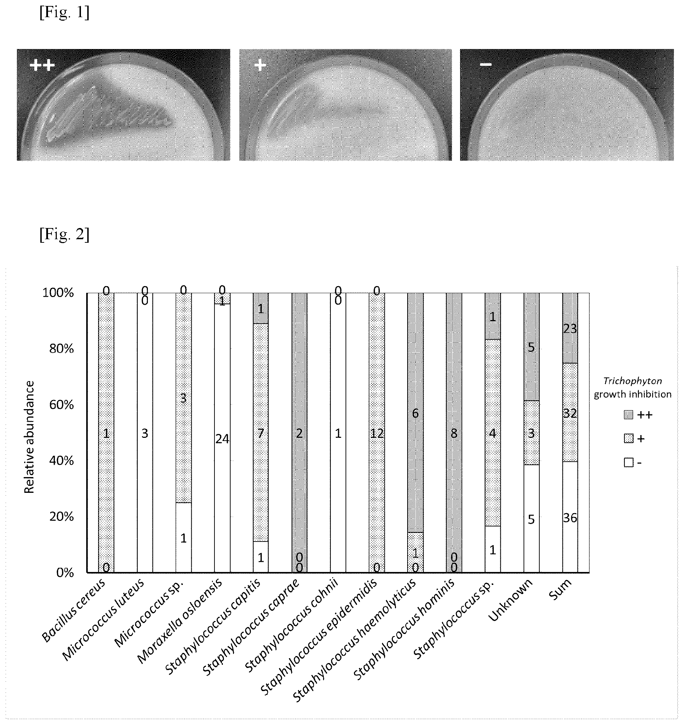

The collected platinum loop to which the bacteria adhered was plated on a fungal mixed culture medium with a streak, and the plate was statically cultured under aerobic conditions at 30°C until turbidity in the agar medium due to growth of Trichophyton was visually observed. After culturing, the growth inhibition region formed in the fungal mixed culture medium was observed, and the anti-ringworm fungal action of the candidate bacteria or known bacteria was evaluated according to the following criteria depending on the size of the growth inhibition region. A typical example of each evaluation is shown in Fig. 1.

++: A clear growth inhibition region of Trichophyton was observed.
+: No clear growth inhibition region was observed, but growth suppression of Trichophyton was observed.
-: No growth suppression of Trichophyton was observed.

First, the results obtained in the known bacteria (32 strains) are shown in the following Table 4.

**[Table 4]**

| No. | Bacterial species | Strains | Results |
|---|---|---|---|
| 1 | Acinetobacter baumannii | ATCC 17978 | - |
| 2 | Corynebacterium glaucum | JCM12208 | - |
| 3 | Corynebacterium jeikeium | ATCC43734 | - |
| 4 | Corynebacterium striatum | NBRC15291 | - |
| 5 | Enterococcus faecalis | ATCC29212 | ++ |
| 6 | Escherichia coli | ATCC25922 | ++ |
| 7 | Micrococcus luteus | ATCC4698 | - |
| 8 | Moraxella atlantae | NBRC14588 | - |
| 9 | Neisseria gonorrhoeae | ATCC19424 | - |
| 10 | Pseudomonas aeruginosa | ATCC27853 | ++ |
| 11 | Staphylococcus aureus | ATCC29213 | - |
| 12 | Staphylococcus epidermidis | ATCC 12228 | + |
| 13 | Staphylococcus haemolyticus | ATCC29970 | + |
| 14 | Staphylococcus hominis | ATCC27844 | ++ |
| 15 | Streptococcus agalactiae | ATCC13813 | - |
| 16 | Streptococcus mitis | JCM12971 | - |
| 17 | Streptococcus pyogenes | ATCC12344 | - |
| 18 | Staphylococcus aureus | ATCC 12600 | + |
| 19 | Staphylococcus epidermidis | ATCC14990 | + |
| 20 | Staphylococcus capitis | ATCC27840 | + |
| 21 | Staphylococcus caprae | ATCC35538 | + |
| 22 | Staphylococcus cohnii | ATCC29974 | - |
| 23 | Staphylococcus haemolyticus | ATCC700564 | ++ |
| 24 | Staphylococcus haemolyticus | ATCC29969 | ++ |
| 25 | Staphylococcus hominis | ATCC27845 | ++ |
| 26 | Staphylococcus hominis | ATCC700236 | ++ |
| 27 | Staphylococcus kloosii | ATCC43959 | + |
| 28 | Staphylococcus lentus | ATCC700403 | - |
| 29 | Staphylococcus lugdunensis | ATCC49576 | + |
| 30 | Staphylococcus lugdunensis | ATCC43809 | + |
| 31 | Staphylococcus warneri | ATCC27836 | ++ |
| 32 | Staphylococcus saprophyticus | ATCC15305 | + |

From the results in Table 4, it can be seen that among the known bacteria (32 strains), 9 strains gave a result of "++", 10 strains gave a result of "+", and 13 strains gave a result of "-".

Further, in the candidate bacteria (91 strains), 23 strains were evaluated as "++", 32 strains as "+", and 36 strains as "-".

### 5. Identification of candidate bacteria

In order to analyze the relationship between the result of the anti-ringworm fungal action by the candidate bacteria and the type of the candidate bacteria, the candidate bacteria were identified. The identification of candidate bacteria was performed by comparing 16s rDNA sequences.

Specifically, according to the attached manual of NucleoSpin Microbial DNA, total DNA was extracted and purified from the candidate bacteria cultured in Anaero Columbia RS Blood Agar Medium, and the DNA concentration and purity were measured using NanoDrop which is an ultra-trace spectrophotometer.

Next, according to the attached manual of Bacterial 16S rDNA PCR Kit, using the total DNA after purification as a template, the 16S rDNA sequence region was amplified by PCR using the attached primer F1 (SEQ ID NO: 19) and primer R2 (SEQ ID NO: 20). The amplified DNA fragment (about 1,500 bp) was purified using NucleoSpin Gel and PCR Clean-up kit, and the DNA concentration and purity were measured using NanoDrop which is an ultra-trace spectrophotometer. It was confirmed that the DNA fragment size according to the theoretical value was amplified by agarose gel electrophoresis.

The amplified DNA fragment (16S rDNA template sample) was mixed with a primer attached to Bacterial 16S rDNA PCR Kit (F1 (SEQ ID NO: 19), F2 (SEQ ID NO: 21) or R2 (SEQ ID NO: 20)), and the target sequence was subjected to Sanger sequence analysis. After the obtained sequence was assembled, the bacterial species of the candidate bacteria were identified by performing BLAST search of NCBI on the nucleotide sequence (about 1,240 to 1,280 bp) in the region between common sequence 1 (AGCTTGC) and common sequence 2 (AAGCTGG) shared by 16s rDNA of genus Staphylococcus. At this time, the nucleotide sequence (about 1,240 to 1,280 bp) of the target region of the 16s rDNA is a part of the DNA fragment (about 1,500 bp) after amplification, and includes common sequence 1 (AGCTTGC) and common sequence 2 (AAGCTGG). In addition, when the obtained sequence had no common sequence 1 (AGCTTGC), substitution was performed in the order of GGTTGG and TGCTTGC, and when the obtained sequence had no common sequence 2 (AAGCTGG), substitution was performed in the order of GAAGTTGG, AAGTCGG, GAAGTCGA, AAGTCAG, GAGCTGG, AAGGGGGG, AAGTCTGG, CAGTCAG, and CCGCTGG, and BLAST search of NCBI was performed on the nucleotide sequence (about 1,240 to 1,280 bp) in the region flanked by the respective common sequences to identify the bacterial species of the candidate bacteria. When the obtained sequence does not contain any one or both of the common sequence 1 (AGCTTGC) or a sequence alternative thereto (GGTTGG, TGCTTGC) and the common sequence 2 (AAGCTGG) or a sequence alternative thereto (GAAGTTGG, AAGTCGG, GAAGTCGA, AAGTCAG, GAGCTGG, AAGGGGGG, AAGTCTGG, CAGTCAG, CCGCTGG), BLAST search of NCBI was performed on the full-length nucleotide sequence of the obtained sequence (amplified DNA fragment (about 1,500 bp)) to identify bacterial species of candidate bacteria.

In identifying the bacterial species of the candidate bacteria, bacterial species showing 99% or more of identity were selected from the top 10 sequences hit by BLAST search. Here, when a plurality of results of different species of the same genus were included in the top 10 bacterial species selected, identification with genus name (Staphylococcus sp. and Micrococcus sp.) was adopted. When a bacterial species satisfying the condition was not identified, it was determined as unknown.

The results of the identified candidate bacteria and the anti-ringworm fungal action are shown in the following Tables 5 and 6.

**[Table 5]**

| No. | Bacterial species | Results | No. | Bacterial species | Results | No. | Bacterial species | Results |
|---|---|---|---|---|---|---|---|---|
| 1 | Staphylococcus epidermidis | + | 21 | Staphylococcus sp. | ++ | 41 | Staphylococcus capitis | + |
| 2 | Staphylococcus hominis | ++ | 22 | Staphylococcus haemolyticus | ++ | 42 | Staphylococcus epidermidis | + |
| 3 | Staphylococcus hominis | ++ | 23 | Staphylococcus haemolyticus | ++ | 43 | Staphylococcus capitis | + |
| 4 | Moraxella osloensis | - | 24 | Staphylococcus haemolyticus | ++ | 44 | unknown | + |
| 5 | Moraxella osloensis | - | 25 | Staphylococcus haemolyticus | ++ | 45 | Staphylococcus hominis | ++ |
| 6 | Moraxella osloensis | - | 26 | unknown | ++ | 46 | unknown | ++ |
| 7 | Moraxella osloensis | - | 27 | Staphylococcus epidermidis | + | 47 | Staphylococcus capitis | + |
| 8 | Moraxella osloensis | - | 28 | Staphylococcus epidermidis | + | 48 | Moraxella osloensis | - |
| 9 | Staphylococcus capitis | - | 29 | Staphylococcus epidermidis | + | 49 | Bacillus cereus | + |
| 10 | Moraxella osloensis | - | 30 | Staphylococcus epidermidis | + | 50 | unknown | ++ |
| 11 | Micrococcus luteus | - | 31 | Staphylococcus epidermidis | + | 51 | Staphylococcus epidermidis | + |
| 12 | Moraxella osloensis | - | 32 | Staphylococcus epidermidis | + | 52 | Moraxella osloensis | - |
| 13 | Moraxella osloensis | - | 33 | Staphylococcus haemolyticus | ++ | 53 | Moraxella osloensis | - |
| 14 | Moraxella osloensis | - | 34 | Staphylococcus epidermidis | + | 54 | Staphylococcus sp. | + |
| 15 | Moraxella osloensis | - | 35 | Staphylococcus haemolyticus | + | 55 | Staphylococcus sp. | + |
| 16 | Moraxella osloensis | - | 36 | unknown | - | 56 | Staphylococcus epidermidis | + |
| 17 | Moraxella osloensis | - | 37 | Staphylococcus hominis | ++ | 57 | Staphylococcus sp. | - |
| 18 | Micrococcus luteus | - | 38 | Staphylococcus capitis | ++ | 58 | Staphylococcus sp. | + |
| 19 | Micrococcus luteus | - | 39 | unknown | ++ | 59 | Staphylococcus capitis | + |
| 20 | Staphylococcus haemolyticus | ++ | 40 | Staphylococcus hominis | ++ | 60 | unknown | ++ |

**[Table 6]**

| No. | Bacterial species | Results | No. | Bacterial species | Results | No. | Bacterial species | Results |
|---|---|---|---|---|---|---|---|---|
| 61 | Staphylococcus caprae | ++ | 71 | Moraxella osloensis | - | 81 | Staphylococcus sp. | + |
| 62 | Staphylococcus hominis | ++ | 72 | Moraxella osloensis | - | 82 | Staphylococcus capitis | + |
| 63 | Staphylococcus caprae | ++ | 73 | Moraxella osloensis | - | 83 | Staphylococcus epidermidis | + |
| 64 | Staphylococcus hominis | ++ | 74 | Staphylococcus hominis | ++ | 84 | unknown | - |
| 65 | Staphylococcus cohnii | - | 75 | Moraxella osloensis | - | 85 | Micrococcus sp. | + |
| 66 | Moraxella osloensis | - | 76 | unknown | - | 86 | Micrococcus sp. | - |
| 67 | Moraxella osloensis | - | 77 | Moraxella osloensis | - | 87 | Moraxella osloensis | + |
| 68 | Moraxella osloensis | - | 78 | Staphylococcus capitis | + | 88 | Micrococcus sp. | + |
| 69 | Moraxella osloensis | - | 79 | unknown | + | 89 | Micrococcus sp. | + |
| 70 | unknown | - | 80 | Staphylococcus capitis | + | 90 | unknown | + |
| | | | | | | 91 | unknown | - |

In Tables 5 and 6, the identified candidate bacteria were sorted into bacterial species, and the results of analyzing the ratio of the anti-ringworm fungal action exhibited by each bacterial species are shown in Fig. 2. From the results in Fig. 2, it was found that 100% and 86% of Staphylococcus hominis and Staphylococcus haemolyticus showed the results of "++", respectively.

In addition, among the 92 candidate bacteria strains, the results of the anti-ringworm fungal action for each separation site of 52 candidate bacteria strains obtained from the malleolus and 39 candidate bacteria strains obtained from the foot sole are shown in the following Table 7.

**[Table 7]**

| Bacterial species | Malleolus | | | | Foot sole | | | | Total of malleolus and foot sole | | | |
|---|---|---|---|---|---|---|---|---|---|---|---|---|
| | Results | | | Total | Results | | | Total | Results | | | Total |
| | ++ | + | - | | ++ | + | - | | ++ | + | - | |
| Bacillus cereus | 0 | 1 | 0 | 1 | 0 | 0 | 0 | 0 | 0 | 1 | 0 | 1 |
| Micrococcus luteus | 0 | 0 | 3 | 3 | 0 | 0 | 0 | 0 | 0 | 0 | 3 | 3 |
| Micrococcus sp. | 0 | 0 | 0 | 0 | 0 | 3 | 1 | 4 | 0 | 3 | 1 | 4 |
| Moraxella osloensis | 0 | 0 | 14 | 14 | 0 | 1 | 10 | 11 | 0 | 1 | 24 | 25 |
| Staphylococcus capitis | 1 | 3 | 1 | 5 | 0 | 4 | 0 | 4 | 1 | 7 | 1 | 9 |
| Staphylococcus caprae | 0 | 0 | 0 | 0 | 2 | 0 | 0 | 2 | 2 | 0 | 0 | 2 |
| Staphylococcus cohnii | 0 | 0 | 0 | 0 | 0 | 0 | 1 | 1 | 0 | 0 | 1 | 1 |
| Staphylococcus epidermidis | 0 | 10 | 0 | 10 | 0 | 2 | 0 | 2 | 0 | 12 | 0 | 12 |
| Staphylococcus haemolyticus | 6 | 1 | 0 | 7 | 0 | 0 | 0 | 0 | 6 | 1 | 0 | 7 |
| Staphylococcus hominis | 5 | 0 | 0 | 5 | 3 | 0 | 0 | 3 | 8 | 0 | 0 | 8 |
| Staphylococcus sp. | 1 | 0 | 0 | 1 | 0 | 4 | 1 | 5 | 1 | 4 | 1 | 6 |
| unknown | 4 | 1 | 1 | 6 | 1 | 2 | 4 | 7 | 5 | 3 | 5 | 13 |
| Total | 17 | 16 | 19 | 52 | 6 | 16 | 17 | 39 | 23 | 32 | 36 | 91 |

From the results of Table 7, in the 52 strains from the malleolus, 33% was "++", 31% was "+", and 37% was "-", and in the 39 strains from the foot sole, 15% was "++", 41% was "+", and 44% was "-". Compared to the foot sole, the malleolus was found to have about twice the proportion of strains exhibiting "++".

### 6. Creation of phylogenetic tree of genus Staphylococcus

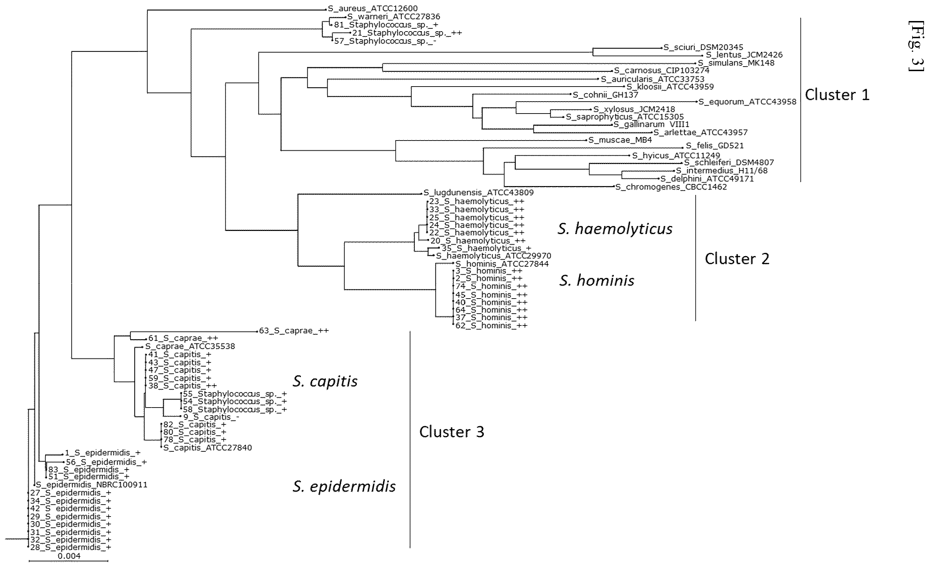

With reference to the phylogenetic tree for 16S rDNA sequences of 27 bacterial species of the genus Staphylococcus in the paper of Ghebremedhin B et al. (J Clin Microbiol, 2008;46:1019-1025: Genetic classification and distinguishing of Staphylococcus species based on different partial gap, 16S rRNA, hsp60, rpoB, sodA, and tuf gene sequences), phylogenetic trees of 27 strains of bacteria of the known genus Staphylococcus (Table 8 below) and 45 strains of bacteria identified as genus Staphylococcus among candidate bacteria (extracts from Tables 5 and 6 are shown in Table 9 below) were created. The created phylogenetic tree is shown in Fig. 3. Note that a CLC sequence viewer (ver. 8.0) was used for creating the phylogenetic tree.

**[Table 8]**

| No. | Bacterial species | Strains |
|---|---|---|
| 1 | Staphylococcus arlettae | ATCC43957 |
| 2 | Staphylococcus aureus | ATCC 12600 |
| 3 | Staphylococcus auricularis | ATCC33753 |
| 4 | Staphylococcus captis | ATCC27840 |
| 5 | Staphylococcus caprae | ATCC35538 |
| 6 | Staphylococcus carnosus | CIP103274 |
| 7 | Staphylococcus chromogenes | CBCC 1462 |
| 8 | Staphylococcus cohnii | GH137 |
| 9 | Staphylococcus delphini | ATCC49171 |
| 10 | Staphylococcus epidermidis | ATCC14990 |
| 11 | Staphylococcus equorum | ATCC43958 |
| 12 | Staphylococcus felis | GD521 |
| 13 | Staphylococcus gallinarum | VIII1 |
| 14 | Staphylococcus haemolyticus | ATCC29970 |
| 15 | Staphylococcus hominis | ATCC27844 |
| 16 | Staphylococcus hyicus | ATCC11249 |
| 17 | Staphylococcus intermedius | H11/68 |
| 18 | Staphylococcus kloosii | ATCC43959 |
| 19 | Staphylococcus lentus | JCM2426 |
| 20 | Staphylococcus lugdunensis | ATCC43809 |
| 21 | Staphylococcus muscae | MB4 |
| 22 | Staphylococcus saprophyticus | ATCC15305 |
| 23 | Staphylococcus schleiferi | DSM4807 |
| 24 | Staphylococcus sciuri | DSM20345 |
| 25 | Staphylococcus simulans | MK148 |
| 26 | Staphylococcus warneri | ATCC27836 |
| 27 | Staphylococcus xylosus | JCM2418 |

**[Table 9]**

| No. | Bacterial species | Results | No. | Bacterial species | Results |
|---|---|---|---|---|---|
| 1 | Staphylococcus epidermidis | + | 42 | Staphylococcus epidermidis | + |
| 2 | Staphylococcus hominis | ++ | 43 | Staphylococcus capitis | + |
| 3 | Staphylococcus hominis | ++ | 45 | Staphylococcus hominis | ++ |
| 9 | Staphylococcus capitis | - | 47 | Staphylococcus capitis | + |
| 20 | Staphylococcus haemolyticus | ++ | 51 | Staphylococcus epidermidis | + |
| 21 | Staphylococcus sp. | ++ | 54 | Staphylococcus sp. | + |
| 22 | Staphylococcus haemolyticus | ++ | 55 | Staphylococcus sp. | + |
| 23 | Staphylococcus haemolyticus | ++ | 56 | Staphylococcus epidermidis | + |
| 24 | Staphylococcus haemolyticus | ++ | 57 | Staphylococcus sp. | - |
| 25 | Staphylococcus haemolyticus | ++ | 58 | Staphylococcus sp. | + |
| 27 | Staphylococcus epidermidis | + | 59 | Staphylococcus capitis | + |
| 28 | Staphylococcus epidermidis | + | 61 | Staphylococcus caprae | ++ |
| 29 | Staphylococcus epidermidis | + | 62 | Staphylococcus hominis | ++ |
| 30 | Staphylococcus epidermidis | + | 63 | Staphylococcus caprae | ++ |
| 31 | Staphylococcus epidermidis | + | 64 | Staphylococcus hominis | ++ |
| 32 | Staphylococcus epidermidis | + | 65 | Staphylococcus cohnii | - |
| 33 | Staphylococcus haemolyticus | ++ | 74 | Staphylococcus hominis | ++ |
| 34 | Staphylococcus epidermidis | + | 78 | Staphylococcus capitis | + |
| 35 | Staphylococcus haemolyticus | + | 80 | Staphylococcus capitis | + |
| 37 | Staphylococcus hominis | ++ | 81 | Staphylococcus sp. | + |
| 38 | Staphylococcus capitis | ++ | 82 | Staphylococcus capitis | + |
| 40 | Staphylococcus hominis | ++ | 83 | Staphylococcus epidermidis | + |
| 41 | Staphylococcus capitis | + | | | |

When the phylogenetic tree in Fig. 3 was compared with the results of the anti-ringworm fungal action of the candidate bacteria, the candidate bacteria were roughly divided into Cluster 1 mainly showing a result of "-", Cluster 2 mainly showing a result of "++", and Cluster 3 mainly showing a result of "+". Among them, Cluster 2 was composed of S. hominis and S. haemolyticus.

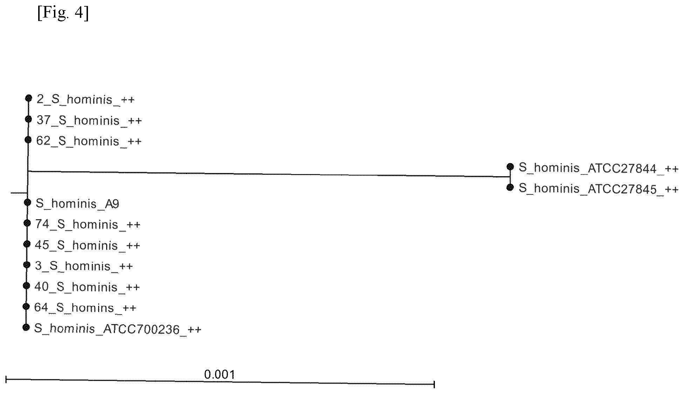

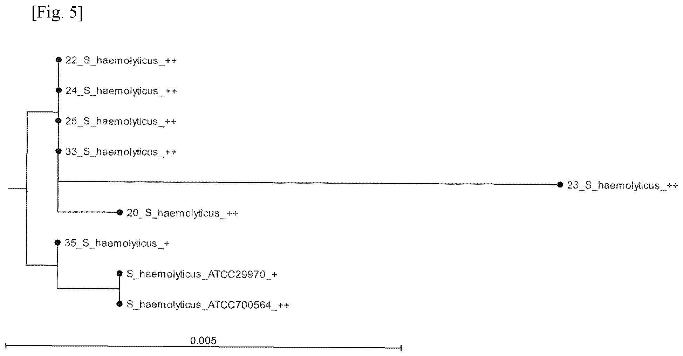

Therefore, phylogenetic trees were created for 6 known strains of bacteria of S. hominis and S. haemolyticus (Table 10 below), and 15 strains of bacteria identified as S. hominis or S. haemolyticus among the candidate bacteria (extracts from Table 9 are shown in Table 11 below). The phylogenetic tree of created S. hominis and S. haemolyticus is shown in Figs. 4 and 5.

**[Table 10]**

| No. | Bacterial species | Strains |
|---|---|---|
| 1 | Staphylococcus hominis | ATCC27844 |
| 2 | Staphylococcus hominis | ATCC27845 |
| 3 | Staphylococcus hominis | ATCC700236 |
| 4 | Staphylococcus hominis | A9 |
| 5 | Staphylococcus haemolyticus | ATCC29970 |
| 6 | Staphylococcus haemolyticus | ATCC700564 |

**[Table 11]**

| No. | Bacterial species | Results |
|---|---|---|
| 2 | Staphylococcus hominis | ++ |
| 3 | Staphylococcus hominis | ++ |
| 37 | Staphylococcus hominis | ++ |
| 40 | Staphylococcus hominis | ++ |
| 45 | Staphylococcus hominis | ++ |
| 62 | Staphylococcus hominis | ++ |
| 64 | Staphylococcus hominis | ++ |
| 74 | Staphylococcus hominis | ++ |
| 20 | Staphylococcus haemolyticus | ++ |
| 22 | Staphylococcus haemolyticus | ++ |
| 23 | Staphylococcus haemolyticus | ++ |
| 24 | Staphylococcus haemolyticus | ++ |
| 25 | Staphylococcus haemolyticus | ++ |
| 33 | Staphylococcus haemolyticus | ++ |
| 35 | Staphylococcus haemolyticus | + |

### 7. 16s rDNA sequence

### (1) 16s rDNA sequence of strains of Staphylococcus hominis

The 16s rDNA sequence of Staphylococcus hominis ATCC27844, which is a known bacterium, was confirmed in the ATCC database, and the nucleotide sequence of the region between the common sequence 1 (AGCTTGC) and the common sequence 2 (AAGCTGG) was confirmed. As a result, the 16s rDNA sequence of Staphylococcus hominis ATCC27844 had the nucleotide sequence of SEQ ID NO: 1 (Staphylococcus hominis ATCC27844 genome position: 716544 to 717811). Since SEQ ID NO: 1 includes common sequence 1 (AGCTTGC) and common sequence 2 (AAGCTGG), the 5'-end of the nucleotide sequence of SEQ ID NO: 1 is common sequence 1 (AGCTTGC), and the 3' end is common sequence 2 (AAGCTGG).

In addition, for the 16s rDNA sequences of the candidate bacteria of Nos. 2, 3, 37, 40, 45, 62, 64, and 74 identified as the strains of Staphylococcus hominis, the nucleotide sequences (about 1,240 to 1,280 bp) of the regions between the common sequence 1 (AGCTTGC) and the common sequence 2 (AAGCTGG) were confirmed in the same manner as in the above "5. Identification of candidate bacteria". As a result, the 16s rDNA sequences of the candidate bacteria of Nos. 3, 40, 45, 64, and 74 had the nucleotide sequence of SEQ ID NO: 5, the 16s rDNA sequence of the candidate bacteria of No. 2 had the nucleotide sequence of SEQ ID NO: 7, the 16s rDNA sequence of the candidate bacteria of No. 37 had the nucleotide sequence of SEQ ID NO: 8, and the 16s rDNA sequence of the candidate bacteria of No. 62 had the nucleotide sequence of SEQ ID NO: 9.

Furthermore, the 16s rDNA sequences of the known bacteria Staphylococcus hominis ATCC27845 and Staphylococcus hominis ATCC700236 were confirmed in the database of ATCC, and the nucleotide sequence of the region between the common sequence 1 (AGCTTGC) and the common sequence 2 (AAGCTGG) was confirmed. In addition, the 16s rDNA sequence of Staphylococcus hominis A9 (ATCC Accession No: PTA-125203) was confirmed in the database of PATRIC, and the nucleotide sequence of the region between common sequence 1 (AGCTTGC) and common sequence 2 (AAGCTGG) was confirmed. As a result, the 16s rDNA sequence of Staphylococcus hominis ATCC27845 had the nucleotide sequence of SEQ ID NO: 1 (Staphylococcus hominis ATCC27845 genome position: 761822 to 763089), and the 16s rDNA sequence of Staphylococcus hominis ATCC700236 and the 16s rDNA sequence of Staphylococcus hominis A9 (ATCC Accession No.: PTA-125203) had the nucleotide sequence of SEQ ID NO: 5 (Staphylococcus hominis ATCC700236 genome position: 849942 to 851209, Staphylococcus hominis A9 genome position: 185 to 1452).

### (2) 16s rDNA sequence of strains of Staphylococcus haemolyticus

The 16s rDNA sequence of Staphylococcus haemolyticus ATCC29970, which is a known bacterium, was confirmed in the ATCC database, and the nucleotide sequence of the region between the common sequence 1 (AGCTTGC) and the common sequence 2 (AAGCTGG) was confirmed. As a result, the 16s rDNA sequence of Staphylococcus haemolyticus ATCC29970 had the nucleotide sequence of SEQ ID NO: 10 (Staphylococcus haemolyticus ATCC29970 genome position:807302 to 808569).

In addition, for the 16s rDNA sequences of the candidate bacteria of Nos. 22 to 25 and 33 and 35 identified as the strain of Staphylococcus haemolyticus, the nucleotide sequences (about 1,240 to 1,280 bp) of the region between common sequence 1 (AGCTTGC) and common sequence 2 (AAGCTGG) were confirmed in the same manner as in the above "5. Identification of candidate bacteria". As a result, the 16s rDNA sequences of the candidate bacteria of Nos. 22, 24, 25, and 33 had the nucleotide sequence of SEQ ID NO: 13, the 16s rDNA sequence of the candidate bacteria of No. 35 had the nucleotide sequence of SEQ ID NO: 14, and the 16s rDNA sequence of the candidate bacteria of No. 23 had the nucleotide sequence of SEQ ID NO: 16. For the 16s rDNA sequence of No. 20 identified as a strain of Staphylococcus haemolyticus, it had common sequence 1 (AGCTTGC) but not common sequence 2 (AAGCTGG). Since the 16s rDNA sequence of No. 20 had an alternative sequence (GAGCTGG) of the common sequence 2, the nucleotide sequence (about 1,267 bp) in the region between the common sequence 1 (AGCTTGC) and the alternative sequence (GAGCTGG) of the common sequence 2 was confirmed. As a result, the 16s rDNA sequence of the candidate bacterium of No. 20 had the nucleotide sequence of SEQ ID NO: 15.

Furthermore, the 16s rDNA sequence of Staphylococcus haemolyticus ATCC700564, which is a known bacterium, was confirmed in the ATCC database, and the nucleotide sequence of the region between the common sequence 1 (AGCTTGC) and the common sequence 2 (AAGCTGG) was confirmed. As a result, the 16s rDNA sequence of Staphylococcus haemolyticus ATCC700564 had the nucleotide sequence of SEQ ID NO: 10 (Staphylococcus haemolyticus ATCC700564 genome position: 214068 to 215335).

The results obtained in the above (1) and (2) are shown in the following Tables 12 and 13.

**[Table 12]**

| Bacterial species | Strains | SEQ ID NO. | Evaluation results of anti-ringworm fungal action |
|---|---|---|---|
| S. hominis | ATCC27844 | SEQ ID NO: 1 | ++ |
| S. hominis | No. 2 | SEQ ID NO: 7 | ++ |
| S. hominis | No. 3 | SEQ ID NO: 5 | ++ |
| S. hominis | No. 37 | SEQ ID NO: 8 | ++ |
| S. hominis | No. 40 | SEQ ID NO: 5 | ++ |
| S. hominis | No. 45 | SEQ ID NO: 5 | ++ |
| S. hominis | No. 62 | SEQ ID NO: 9 | ++ |
| S. hominis | No. 64 | SEQ ID NO: 5 | ++ |
| S. hominis | No. 74 | SEQ ID NO: 5 | ++ |
| S. hominis | ATCC27845 | SEQ ID NO: 1 | ++ |
| S. hominis | ATCC700236 | SEQ ID NO: 5 | ++ |
| S. hominis | A9 | SEQ ID NO: 5 | ND |
| S. haemolyticus | ATCC29970 | SEQ ID NO: 10 | + |
| S. haemolyticus | No. 20 | SEQ ID NO: 15 | ++ |
| S. haemolyticus | No. 22 | SEQ ID NO: 13 | ++ |
| S. haemolyticus | No. 23 | SEQ ID NO: 16 | ++ |
| S. haemolyticus | No. 24 | SEQ ID NO: 13 | ++ |
| S. haemolyticus | No. 25 | SEQ ID NO: 13 | ++ |
| S. haemolyticus | No. 33 | SEQ ID NO: 13 | ++ |
| S. haemolyticus | No. 35 | SEQ ID NO: 14 | + |
| S. haemolyticus | ATCC700564 | SEQ ID NO: 10 | ++ |

| | | | |
|---|---|---|---|
| *ND: no data | | | |

**[Table 13]**

| SEQ ID NO. | Mutation site based on SEQ ID NO: 1 or 10 |
|---|---|
| SEQ ID NO: 1 | - |
| SEQ ID NO: 5 | 45G>A |
| SEQ ID NO: 7 | 45G>A, 488delA |
| SEQ ID NO: 8 | 45G>A, 1182_1183insA |
| SEQ ID NO: 9 | 45G>A, 1235_1236insG |
| SEQ ID NO: 10 | - |
| SEQ ID NO: 13 | 398A>G, 952C>T |
| SEQ ID NO: 14 | 398A>G |
| SEQ ID NO: 15 | 398A>G, 952C>T, 1186C>T |
| SEQ ID NO: 16 | 398A>G, 952C>T, 1117_1118insG, 1195_1196insC, 1210delA, 1219delA, 1225delT, 1234_1235insG, 1263delA |

In Table 13, SEQ ID NOs: 5 and 7 to 9 are based on SEQ ID NO: 1, and SEQ ID NOs: 13 to 16 are based on SEQ ID NO: 10. For example, "45G> A" described in SEQ ID NO: 5 means that the G (guanine) at position 45 of SEQ ID NO: 1 is mutated to A (adenine). Also, for example, "488delA" described in SEQ ID NO: 7 means that A (adenine) at position 488 of SEQ ID NO: 1 is deleted. Furthermore, for example, "1182 _1183insA" described in SEQ ID NO: 8 means that A (adenine) is inserted between positions 1182 and 1183 of SEQ ID NO: 1.

### (3) 16s rDNA sequence of the strain of Staphylococcus warneri

The 16s rDNA sequence of Staphylococcus warneri ATCC27836, which is a known bacterium, was confirmed in the ATCC database, and the nucleotide sequence of the region between the common sequence 1 (AGCTTGC) and the common sequence 2 (AAGCTGG) was confirmed. As a result, the 16s rDNA sequence of Staphylococcus warneri ATCC27836 had the nucleotide sequence of SEQ ID NO: 17 (Staphylococcus warneri ATCC27836 genome position: 725369 to 726636).

From the results of 7(1) to 7(3), it is considered that S. hominis having 16s rDNA containing a nucleotide sequence having SEQ ID NO: 1 or 95% or more of identity to SEQ ID NO: 1, S. haemolyticus having 16s rDNA containing a nucleotide sequence having SEQ ID NO: 10 or 95% or more of identity to SEQ ID NO: 10, and S. warneri having 16s rDNA containing a nucleotide sequence having SEQ ID NO: 17 or 95% or more of identity to SEQ ID NO: 17 have an anti-ringworm fungal action.

### 8. Effects on other Trichophyton

In the above 4 to 7, a fungal mixed culture medium containing Trichophyton rubrum ATCC28188, which is a Trichophyton, was used as an evaluation medium (see above "3. Preparation of culture medium for evaluation"). Here, the effect on other Trichophyton (Trichophyton other than T. rubrum ATCC28188) was evaluated.

### (1) Preparation of fungal mixed culture medium containing other Trichophyton

Except that beads of Trichophyton rubrum ATCC22402, beads of Trichophyton mentagrophytes TIMM2789, beads of Trichophyton mentagrophytes ATCCMYA-4439, beads of Trichophyton tonsurans ATCC56186, or beads of Trichophyton tonsurans ATCC28942 were used in place of the Trichophyton rubrum ATCC28188, a fungal mixed culture medium containing 5 types of Trichophyton was produced in the same manner as in the above "3. Preparation of culture medium for evaluation".

### (2) Evaluation of anti-ringworm fungal action on other Trichophyton

For the known bacteria S. hominis ATCC27844, S. hominis ATCC27845, S. haemolyticus ATCC29969, S. haemolyticus ATCC700564, and S. warneri ATCC27836 for which the anti-ringworm fungal action on Trichophyton rubrum ATCC28188 was evaluated, the anti-ringworm action was evaluated using a fungal mixed culture medium containing the above 5 types of Trichophyton.

The evaluation method was performed in the same manner as in the above "4. Evaluation of anti-ringworm fungal action". The obtained results are shown in Table 13 below together with the results of the anti-ringworm fungal action on T. rubrum ATCC28188.

**[Table 14]**

| Bacterial species | Strains | SEQ ID NO. | Evaluation results of anti-ringworm fungal action | | | | | |
|---|---|---|---|---|---|---|---|---|
| | | | T. rubrum | | T. mentagrophytes | | T. tonsurans | |
| | | | ATCC 28188 | ATCC 22402 | TIMM 2789 | ATCCMY A-4439 | ATCC 56186 | ATCC 28942 |
| S. hominis | ATCC27844 | SEQ ID NO: 1 | ++ | ++ | ++ | ++ | ++ | ++ |
| S. hominis | ATCC27845 | SEQ ID NO: 1 | ++ | ++ | ++ | ++ | ++ | ++ |
| S. haemolyticus | ATCC29969 | SEQ ID NO: 10 | ++ | ++ | ++ | ++ | ++ | ++ |
| S. haemolyticus | ATCC700564 | SEQ ID NO: 10 | ++ | ++ | ++ | ++ | ++ | ++ |
| S. warneri | ATCC27836 | SEQ ID NO: 17 | ++ | ++ | ++ | ++ | ++ | ++ |

From the results in Table 14, it can be seen that the same anti-ringworm fungal action as that of T. rubrum ATCC28188 was exhibited also in 5 other Trichophyton.

## Claims

1. A composition for treating ringworm, comprising at least one selected from the group consisting of a strain of Staphylococcus hominis, a strain of Staphylococcus haemolyticus, and a strain of Staphylococcus warneri.

2. The composition for treating ringworm according to claim 1, comprising the strain of Staphylococcus hominis.

3. The composition for treating ringworm according to claim 2, wherein the strain of Staphylococcus hominis has 16s rDNA including a nucleotide sequence of SEQ ID NO: 1 or 16s rDNA including a nucleotide sequence having 95% or more of identity to SEQ ID NO: 1 and has an anti-ringworm action.

4. The composition for treating ringworm according to claim 2 or 3, wherein the strain of Staphylococcus hominis includes at least one selected from the group consisting of Staphylococcus hominis ATCC27844, Staphylococcus hominis ATCC27845, Staphylococcus hominis ATCC700236, and Staphylococcus hominis A9 (ATCC Accession No.: PTA-125203).

5. The composition for treating ringworm according to any one of claims 1 to 4, comprising the strain of Staphylococcus haemolyticus.

6. The composition for treating ringworm according to claim 5, wherein the strain of Staphylococcus haemolyticus has a 16s rDNA including a nucleotide sequence of SEQ ID NO: 10 or a 16s rDNA including a nucleotide sequence having 95% or more of identity to SEQ ID NO: 10, and has an anti-ringworm action.

7. The composition for treating ringworm according to claim 5 or 6, wherein the strain of Staphylococcus haemolyticus includes at least one selected from the group consisting of Staphylococcus haemolyticus ATCC29970, Staphylococcus haemolyticus ATCC700564, and Staphylococcus haemolyticus ATCC29969.

8. The composition for treating ringworm according to any one of claims 1 to 7, comprising the strain of Staphylococcus warneri.

9. The composition for treating ringworm according to claim 8, wherein the strain of Staphylococcus warneri has 16s rDNA including a nucleotide sequence of SEQ ID NO: 17 or 16s rDNA including a nucleotide sequence having 95% or more of identity to SEQ ID NO: 17, and has an anti-ringworm action.

10. The composition for treating ringworm according to claim 8 or 9, wherein the strain of Staphylococcus warneri includes Staphylococcus warneri ATCC27836.

11. The composition for treating ringworm according to any one of claims 1 to 10, wherein a causative fungus of the ringworm includes a fungus of the genus Trichophyton.

12. The composition for treating ringworm according to claim 11, wherein the causative fungus of the ringworm includes at least one selected from the group consisting of a fungal strain of Trichophyton rubrum, a fungal strain of Trichophyton mentagrophytes, and a fungal strain of Trichophyton tonsurans.

13. The composition for treating ringworm according to any one of claims 1 to 12, wherein the composition is an external preparation.

14. A method for producing a composition for treating ringworm, the method comprising:
a step (1) of collecting and culturing a bacterial flora from at least one of a malleolus and a foot sole of a human;
a step (2) of isolating and culturing the bacterial flora cultured in the step (1) to obtain a candidate bacterium;
a step (3) of evaluating an anti-ringworm fungal action of the candidate bacterium to identify a bacterium having an anti-ringworm fungal action; and
a step (4) of preparing a composition for treating ringworm including the bacterium having an anti-ringworm fungal action;
wherein the bacterium having an anti-ringworm fungal action includes at least one selected from the group consisting of a strain of Staphylococcus hominis, a strain of Staphylococcus haemolyticus, and a strain of Staphylococcus warneri.
